# EUROPEAN PATENT SPECIFICATION

(11) **EP 3 894 429 B1**
(45) Date of publication and mention of the grant of the patent: **21.02.2024**
(21) Application number: 19836764.1
(22) Date of filing: 13.12.2019
(51) Int. Cl.: C07K 14/005, C12N 7/00, A61K 39/12

(54) **CRIMEAN-CONGO HEMORRHAGIC FEVER VIRUS REPLICON PARTICLES AND USE THEREOF**
REPLIKON-PARTIKEL DES KRIM-KONGO-FIEBERVIRUS UND DEREN VERWENDUNG
PARTICULES DE RÉPLICON DU VIRUS DE LA FIÈVRE HÉMORRAGIQUE DE CRIMÉE-CONGO ET UTILISATION CORRESPONDANTE

(30) Priority: 14.12.2018 US 201862780098 P
(43) Date of publication of application: 20.10.2021
(73) Proprietor: University of Georgia Research Foundation, Inc., Athens, GA 30602 (US); The United States of America, as represented by the Secretary, Department of Health and Human Services, Bethesda, MD 20892-7660 (US)
(72) Inventor: BERGERON, Eric, Atlanta, GA 30333 (US); PEGAN, Scott, D., Riverside, CA 92506 (US); WELCH, Stephen, R., Atlanta, GA 30333 (US); SCHOLTE, Florine, E. M., Atlanta, GA 30333 (US); SPIROPOULOU, Christina, F., Atlanta, GA 30333 (US); NICHOL, Stuart, T., Atlanta, GA 30333 (US); SPENGLER, Jessica, R., Atlanta, GA 30329-4027 (US)
(74) Representative: Forresters IP LLP
(86) International application number: PCT/US2019/066304
(87) International publication number: WO 2020/123989

(56) References cited:
- WO-A1-2014/028511
- WO-A2-2013/192144
- SCHOLTE F E M ET AL: "Single-dose replicon particle vaccine provides complete protection against Crimean-Congo hemorrhagic fever virus in mice", EMERGING MICROBES & INFECTIONS, vol. 8, no. 1, 4 January 2019 (2019-01-04) , pages 575-578, XP055676872, ISSN: 2222-1751, DOI: 10.1080/22221751.2019.1601030
- SPENGLER J R ET AL: "Heterologous protection against Crimean-Congo hemorrhagic fever in mice after a single dose of replicon particle vaccine", ANTIVIRAL RESEARCH, ELSEVIER BV, NL, vol. 170, 1 August 2019 (2019-08-01), XP085839660, ISSN: 0166-3542, DOI: 10.1016/J.ANTIVIRAL.2019.104573 [retrieved on 2019-08-01]
- ZIVCEC M ET AL: "Molecular Insights into Crimean-Congo Hemorrhagic Fever Virus", VIRUSES, vol. 8, no. 4, 21 April 2016 (2016-04-21), page 106, XP055676857, CH ISSN: 1999-4915, DOI: 10.3390/v8040106

## Description

### CROSS REFERENCE TO RELATED APPLICATIONS

This claims the benefit of U.S. Application No. 62/780,098, filed December 14, 2018.

### STATEMENT OF GOVERNMENT SUPPORT

This invention was made with Government support under grant no. R01AI109008 from the National Institutes of Health, National Institute of Allergy and Infectious Disease. The United States Government has certain rights in the invention.

### FIELD OF THE DISCLOSURE

This disclosure concerns Crimean-Congo Hemorrhagic Fever (CCHF) virus, replicon particles (VRPs), methods of making such VRPs, immunogenic compositions, and use of the immunogenic compositions in methods for inducing an immune response to a CCHF virus.

### BACKGROUND

Crimean-Congo hemorrhagic fever virus (CCHFV) is a negative (-) sense RNA virus of the Nairoviridae family (order Bunyavirales). It causes severe hemorrhages in humans, but no overt disease in animals. This tick-borne virus is widely distributed across Africa, Europe, the Middle East, and Asia. With mortality rates as high as 80% and with no FDA-approved vaccines or therapeutics, CCHF virus is considered a dangerous emerging human pathogen (Weber and Mirazimi, Cytokine Growth Factor Rev 19:395-404).

Surprisingly, a viral homologue of an ovarian tumor domain protease (OTU) was identified within the L-protein of CCHF virus and 39 other known nairoviruses, including the economically damaging Nairobi sheep disease virus, as well as Issyk-kul, Dugbe, and Erve (ERVV) viruses, which cause human disease of varying severity (Capodagli et al., 2013. Journal of Virology 87:3815-3827; Capodagli et al. 2011. J Virol 85:3621-30; Emerg Infect Dis 15:147-54; Dilcher et al., 2012. Virus Genes doi:10.1007/s11262-012-0796-8; Frias-Staheli et al., 2007. Cell Host & Microbe 2:404-416; Peyrefitte et al., 2010. J Gen Virol 91: 189-98). The genome of CCHF virus, like other nairoviruses, consists of 3 negative (-)-sense RNA segments: small (S), medium (M), and large (L). Despite co-localizing with the RNA-dependent RNA polymerases (RdRps), these viral OTUs (vOTUs) are not strictly required for RdRp activity, but instead have been shown in recombinant systems to reverse post-translational modifications of host proteins by ubiquitin (Ub) and Ub-like interferon-simulated gene product 15 (ISG15) (Capodagli et al., 2013. Journal of Virology 87:3815-3827; Bergeron et al., 2010. J Virol 84:216-26. Scholte et al., 2017. Cell Rep 20:2396-2407). Conjugation of Ub and ISG15 to host proteins plays a critical role in regulating antiviral proteins of the interferon (IFN) type 1 response (Weber and Mirazimi, Cytokine Growth Factor Rev 19:395-404; Scholte et al., 2017. Cell Rep 20:2396-2407; Zhao et al., 2005. Proc Natl Acad Sci U S A 102: 10200-5; Speer et al., 2016. Nat Commun 7:11496; Hu and Sun, 2016. Cell Res 26:457-83; Ketscher et al., 2015. Proc Natl Acad Sci U S A 112:1577-82; Niemeyer et al., 2018. PLoS Pathog 14:e1007296). As a result, vOTUs are considered a nairovirus virulence factor because of their role in subverting the host antiviral response.

The continuous spread of the often-fatal CCHF virus to new regions, coupled with the absence of efficacious treatments, has accentuated the need for human and animal vaccines (Hinkula et al., 2017. J Virol 91; Dowall et al., 2017. Vaccine doi:10.1016/j.vaccine.2017.05.031; Canakoglu et al., 2015. PLoS Negl Trop Dis 9:e0003579; Mousavi-Jazi et al., 2011. Scand J Infect Dis 43:225-9; Papa et al., 2011. Scand J Infect Dis 43:225-9). Following the Ebola virus outbreak in Western Africa in 2013-2016, CCHF virus is included on WHO's Research and Development Blueprint list of infectious agents critically needing effective prophylaxis and therapeutics to prevent major outbreaks. A need remains for new vaccines that can be used to induce a protective immune response to CCHF virus.

SCHOLTE F E M ET AL, Single-dose replicon particle vaccine provides complete protection against Crimean-Congo hemorrhagic fever virus in mice, EMERGING MICROBES & INFECTIONS, 8:1, 575-578 (2019), discloses a CCHF VRP vaccine candidate.

SPENGLER J R ET AL, "Heterologous protection against Crimean-Congo hemorrhagic fever in mice after a single dose of replicon particle vaccine", ANTIVIRAL RESEARCH, ELSEVIER BV, NL, vol. 170, 1-5 (2019), discloses a single dose VRP vaccine regimen for CCHF.

ZIVCEC M ET AL, "Molecular Insights into Crimean-Congo Hemorrhagic Fever Virus", VIRUSES, vol. 8(4), 106, 1-21 (2016), provides a review of molecular advances in CCHFV-related research.

PCT publication number WO2014028511 discloses genetically modified nairoviruses that possess a viral ovarian tumor protease with decreased ability to remove ubiquitin (Ub) and ISG15 tags that the human organism uses to label proteins for removal.

PCT publication number WO2013192144 discloses a system for the reverse genetics generation of a Rift Valley fever (RVF) virus replicon particle (VRPRVF) vaccine candidate.

### SUMMARY OF THE DISCLOSURE

Disclosed is the use of a reverse genetics-based approach to generate CCHF virus replicon particles (CCHF VRPs). VRPs undergo one full round of replication, but do not spread because they lack the glycoprotein precursor (GPC)-encoding M genome segment. Thus, these VRPs are not natural. VRPs can only be amplified by supplying GPC in trans. Unlike most vaccine approaches tested, VRPs do not solely rely on the expression of the hypervariable GPC gene, which is unlikely on its own, to confer adequate protection against divergent strains of a CCHF virus. Instead, VRPs abundantly produce the L protein and nucleoprotein, the most conserved CCHF viral proteins. Consequently, CCHF VRPs build protective immunity against genetically divergent strains of the CCHF virus. In some embodiments of the use in the methods, only one dose can produce a protective immune response.

The invention is defined in the claims. The invention provides CCHF VRPs that include a CCHF virus Gn and Gc glycoproteins; CCHF virus L protein; CCHF virus nucleoprotein; a CCHF virus L genome segment; and a CCHF virus S genome segment, wherein the CCHF VRP a) does not contain a CCHF virus M genome segment or b) encodes a domain of GPC but not a full-length GPC. In specific non-limiting examples, the CCHF VRP encodes the domain of the GPC, wherein the domain is a mucin-like domain, GP38 domain, mucin-like+GP38 domain, or an NsM, Gn, Gc receptor binding domain. In some embodiments, the L genome segment encodes a viral ovarian tumor domain protease (vOTU) comprising one or more mutations, wherein the one or more mutations disrupt vOTU deubiquitinase activity and/or interferon-simulated gene product 15 (ISG15) activity. In some embodiments, the S genome segment comprises a CCHF virus nucleoprotein open reading frame (ORF) and a heterologous ORF. In some examples, the heterologous ORF encodes a portion of CCHFV virus GPC, an antigen of interest, or a reporter protein (such as a fluorescent protein). In some non-limiting examples, the heterologous ORF encodes one or more non-structural proteins (GP160, G85, GP38 and Nsm) but not the complete the Gn and/or Gc glycoprotein.

Also provided as part of the invention is a method of producing CCHF VRP, the method includes transfecting a host cell with: a plasmid comprising an antigenomic copy of a CCHF virus L segment; a plasmid comprising an antigenomic copy of a CCHF virus S segment; a plasmid encoding CCHF virus GPC; a plasmid encoding a CCHF virus nucleoprotein; a plasmid encoding a CCHF virus L protein; optionally a plasmid encoding the bacteriophage T7 RNA polymerase, when a T7 promoter is utilized, and culturing the cells for a period of time sufficient to produce CCHF VRP comprising a CCHF virus L genome segment, a CCHF virus S genome segment, the GPC, or a portion thereof, the nucleoprotein and the L protein. In some examples, the method further includes collecting the CCHF VRP from the cell culture supernatant. In some examples, the method further includes amplifying the CCHF VRP in a susceptible cell line expressing CCHF virus GPC in trans.

Further provided as part of the invention are immunogenic compositions that the CCHF VRP of the invention, and a pharmaceutically acceptable carrier.

Also provided as part of the invention is the immunogenic composition of the invention for use in a method of eliciting an immune response against CCHF virus or a CCHF viral protein in a subject or of immunizing a subject against CCHF virus infection by administering to the subject an effective amount of the immunogenic composition of the invention.
In some examples, the CCHF viral protein is a CCHF virus nucleoprotein or a CCHF virus L protein.

The foregoing and other features and advantages of the invention will become more apparent from the following detailed description of several embodiments which proceeds with reference to the accompanying figures.

### BRIEF DESCRIPTION OF THE FIGURES

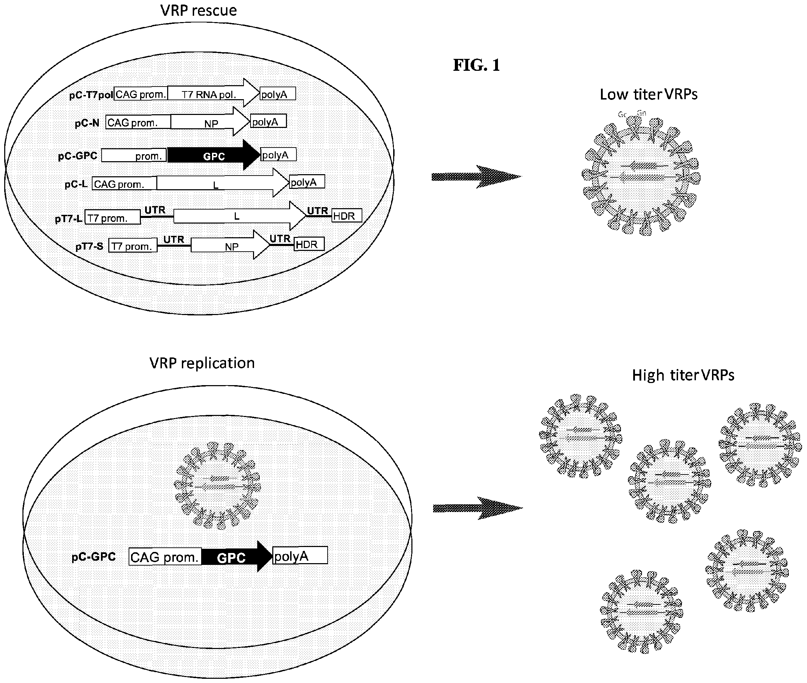

**FIG.** 1 shows a schematic of CCHF VRP rescue using a plasmid encoding T7 RNA polymerase (pC-T7pol), a plasmid encoding nucleoprotein (pC-N), a plasmid encoding GPC (pC-GPC), a plasmid encoding L protein (pC-L), a plasmid containing an antigenomic copy of the L segment (pT7-L) and a plasmid containing an antigenomic copy of the S segment (pT7-S). Transfection of cells with these six plasmids produces low titer VRPs (top). High titer VRPs can be produced by providing a plasmid encoding GPC *in trans* (bottom).

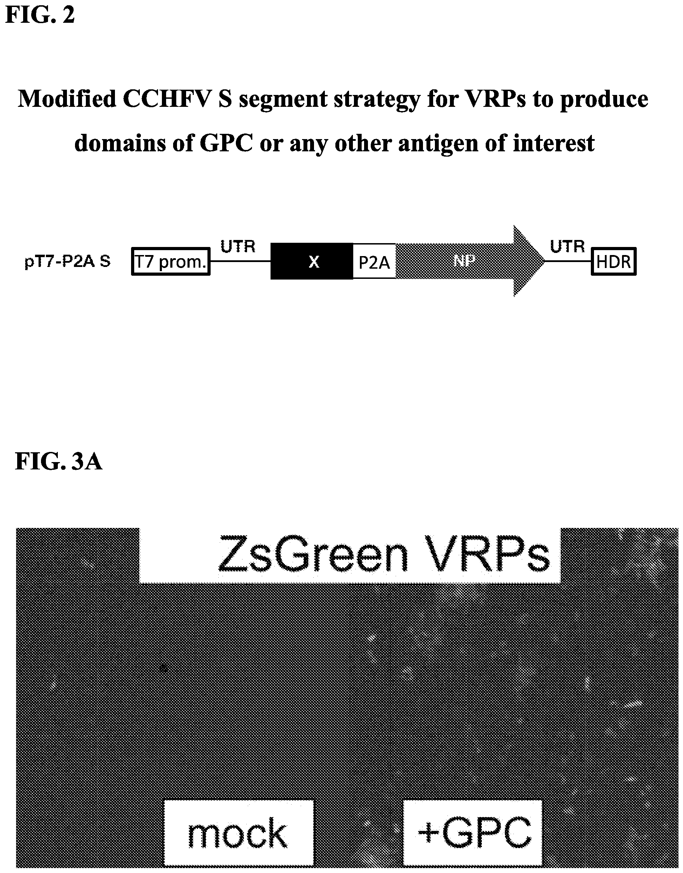

**FIG. 2** is a schematic of a modified CCHFV S segment for expressing domains of GPC or another antigen of interest. An antigen coding sequence ("X") and the coding sequence of a self-cleaving peptide, such as porcine teschovirus-1 2A (P2A), are cloned in-frame with nucleoprotein (NP). This strategy allows the VRPs to produce an antigen of interest and nucleoprotein from a single S segment.

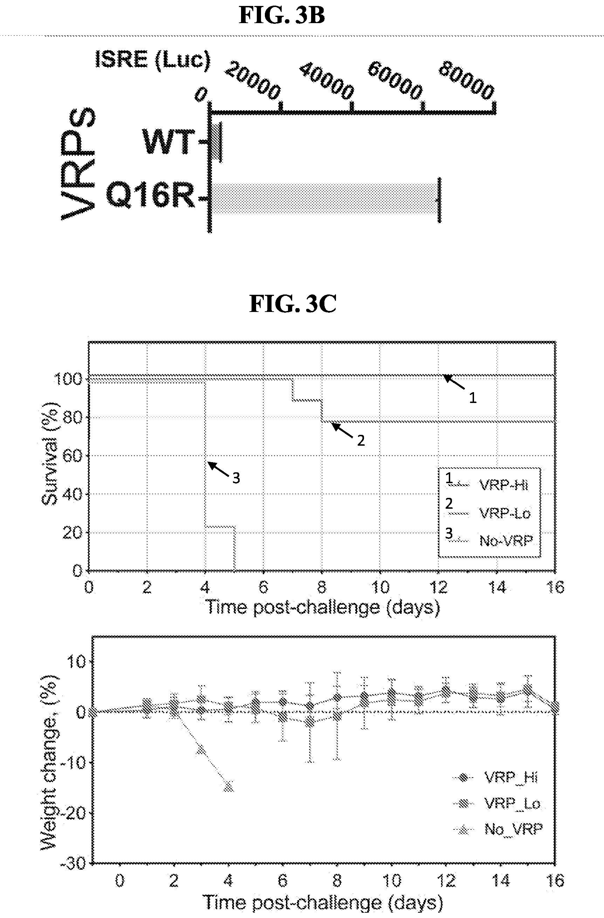

**FIGS. 3A-3C****.** VRP Data. (FIG. 3A). Propagation of ZsGreen VRPs requires GPC expression. (FIG. 3B) Immune response in THP-1 cells infected with VRPs containing either wt or Q16R vOTUs. (FIG. 3C) Survival curve and weight of immunized mice after subcutaneous challenge with CCHFV.

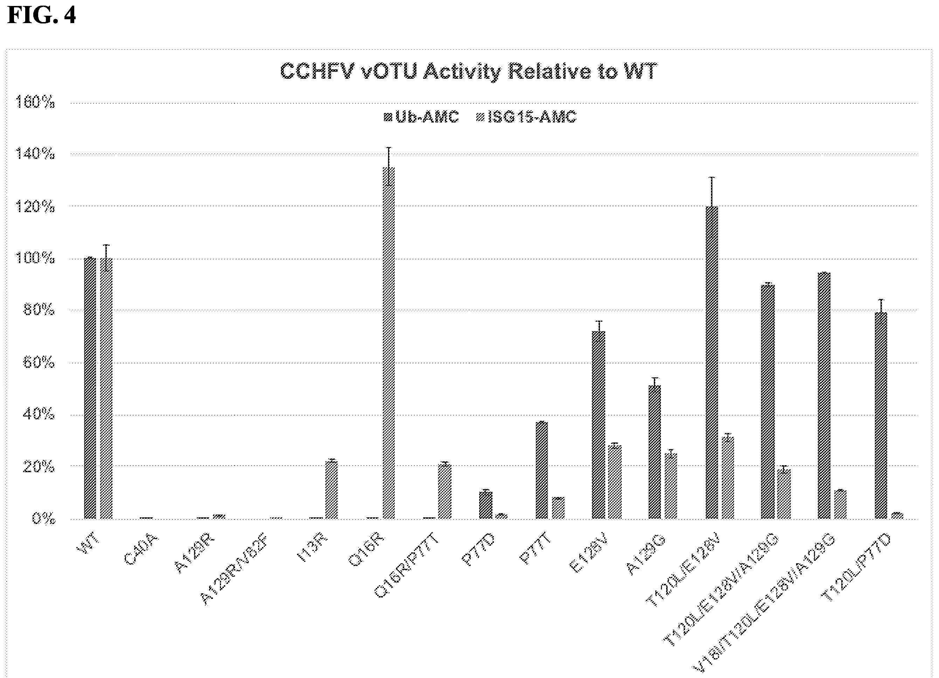

**FIG. 4** shows CCHF vOTU enzymatic data pertaining to the ability of certain mutations to attenuate deubiquitinase and deISGylase activity.

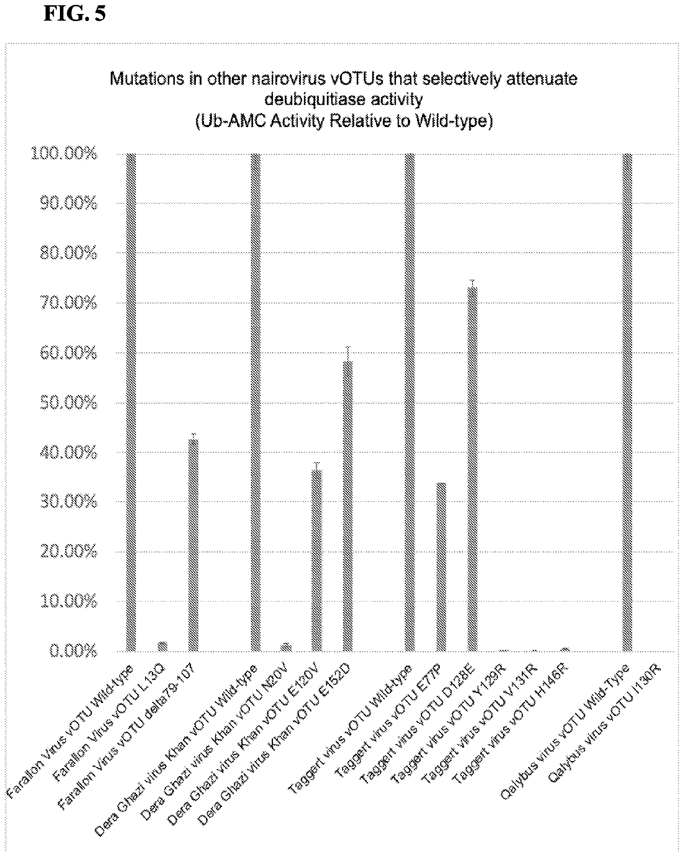

**FIG. 5** is a bar graph showing the mutations in nairovirus vOTUs that selectively attenuate deubiquitase activity. The Ub-AMC activity relative to wild-type is shown. These vOTU mutants are of use in the VRP disclosed herein.

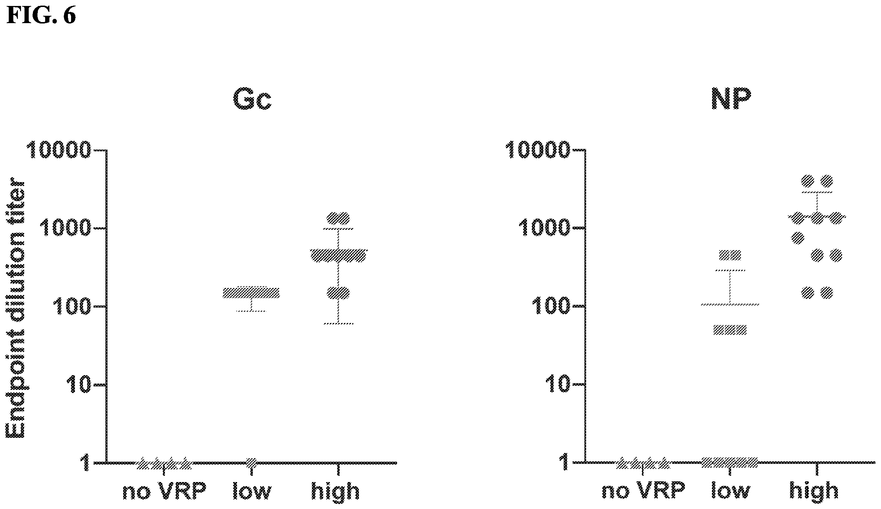

**FIG. 6** is ELISA data, showing immunogenicity of the CCHF VRP. Prior to CCHFV challenge, sera from mice vaccinated with the VRPs were collected 32 days post-vaccination. Nucleoprotein (NP) and Gc specific IgG titers from sera were obtained by endpoint dilution using NP and Gc ELISAs

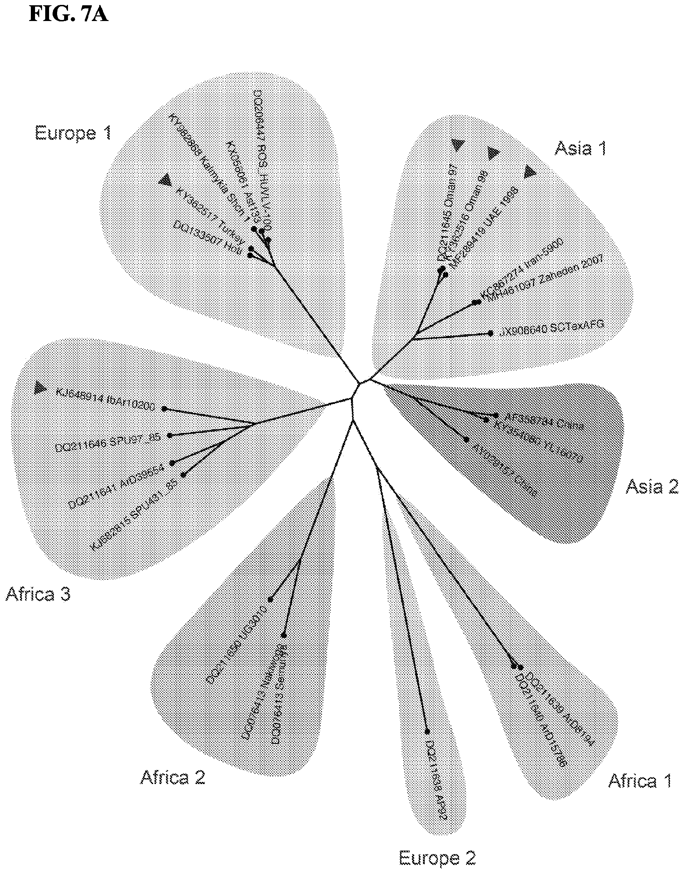

**FIGS. 7A-7E** are plots comparing infection with diverse CCHFV strains in IFNAR^{-/-} mice. (A) Phylogenetic designation by clade of CCHFV (listed by GENBANK^{™} Accession No. and strain name) based on full-length S-genome segment, see the examples. Strains used for in vivo comparison in mice are indicated by black arrowheads. (B) Weight change and (C) survival in mice inoculated SC with a target dose of 1 × 10² TCID₅₀ of indicated CCHFV strains. (D) RT-PCR analyses of viral RNA (S segment) in blood and tissues, and (E) antibody activity units (AAU) of anti-NP IgM or IgG, and anti-Gc IgG in plasma collected when mice reached end-point criteria (open symbols) or at completion of the study (closed symbols; 21 dpi). Time post-infection of sampling, outcome, and corresponding antibody levels for individual mice is provided in the Examples section.

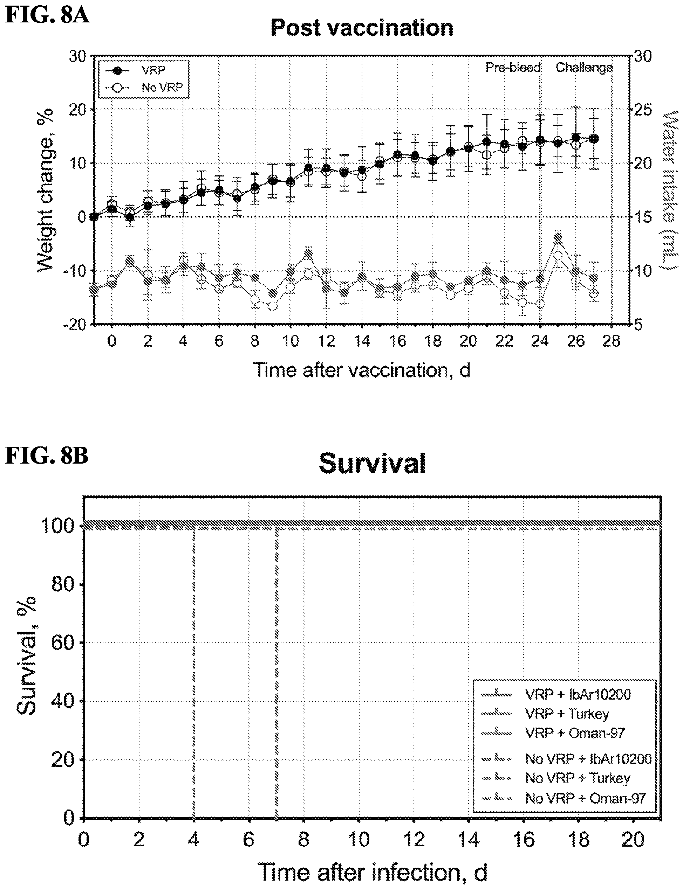

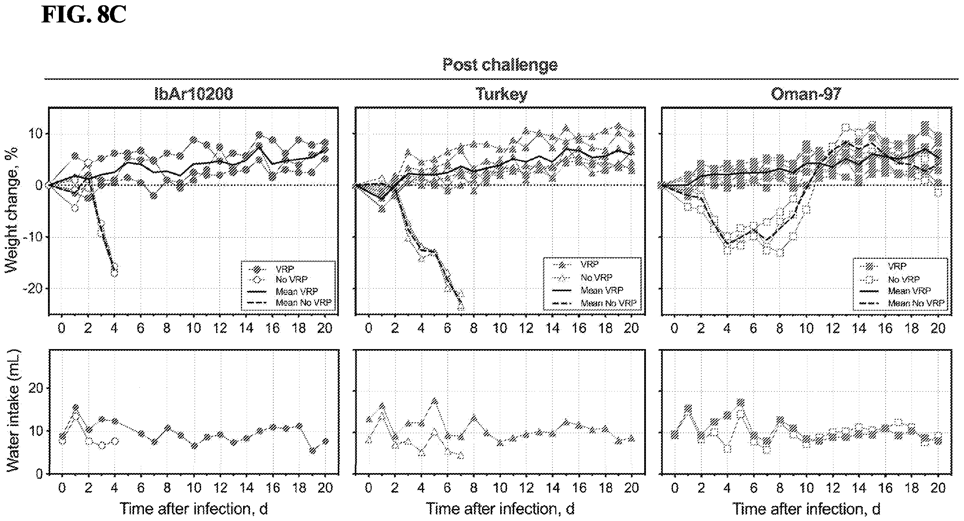

**FIGS. 8A-8D** are graphs showing the clinical outcome of heterologous CCHFV challenge following VRP vaccination. (A) Weight change in mice following VRP vaccination (1 × 10⁵ TCID₅₀). (B) Survival in mice challenged SC with a target dose of 100 TCID₅₀ of indicated CCHFV strains 28 days after VRP vaccination. (C) Post-challenge weight change and water intake. (D) AAU of anti-NP IgM or IgG, and anti-Gc IgG in plasma collected when mice reached end-point criteria (open symbols) or at completion of the study (closed symbols; 21 days post challenge). Time post-infection of sampling, outcome, and corresponding antibody levels for individual mice is provided in the Examples section.

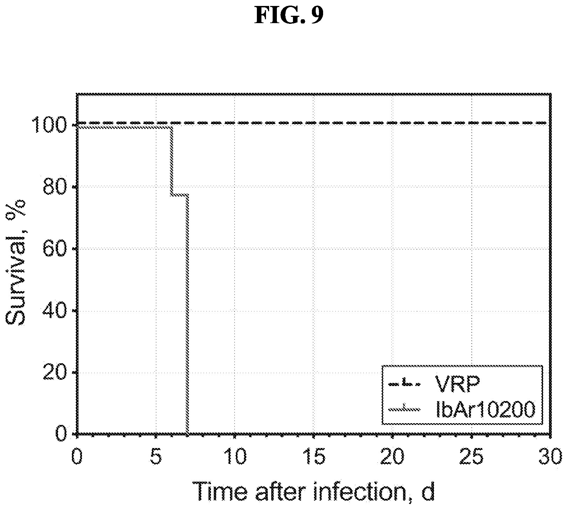

**FIG. 9** shows survival of suckling mice inoculated with CCHFV or VRP. One- or two-day-old CD-1 suckling mice (Charles River; 022CD1) were inoculated intracranially with 1.31 × 10² TCID₅₀ of recombinant IbAR10200 CCHFV (n = 25) or ~100-fold higher dose of CCHFV VRP (1.26 × 10⁴ TCID₅₀; n =11), and monitored daily for clinical signs. Suckling mice with abnormal development prior to inoculation or documented failure to thrive were omitted from analysis (n = 2, CCHFV; n = 1, VRP). All suckling mice inoculated with CCHFV succumbed to acute onset disease by 7 dpi. No clinical signs were observed in VRP-inoculated mice; all VRP inoculated mice survived until study completion (19 dpi).

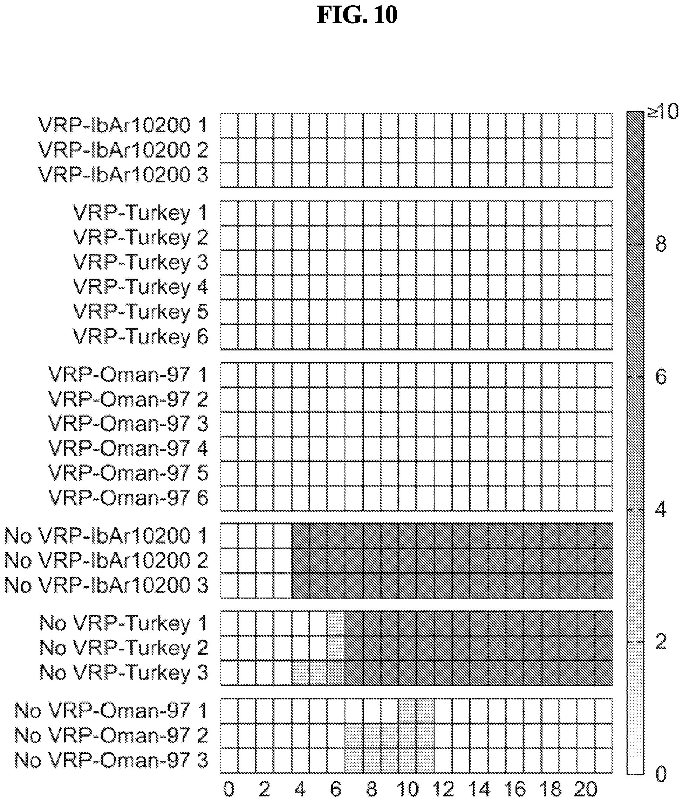

**FIG. 10** shows the clinical scores in VRP-vaccinated IFNAR^{-/-} mice after heterologous CCHFV challenge. Mice were challenged SC with a target dose of 100 TCID₅₀ of indicated CCHFV strains 28 days after VRP vaccination. Mice were scored based on 14 parameters: 2 points each for quiet, dull, responsive (QDR) disposition, hunched back, or ruffled coat; 3 points each for dehydration or abnormal huddling/hypoactivity; 5 points each for presence of neurological signs (ataxia, circling, tremors, or paresis), abnormal breathing, or anemia; 7 points for weight loss of >20% from baseline (-1 dpi); 10 points each for inability to bear weight, paralysis, frank hemorrhage/bleeding, moribund state, or weight loss of >25% from baseline. Animals were humanely euthanized when end-point criteria were reached (clinical score ≥ 10), or at study completion (21 days post challenge).

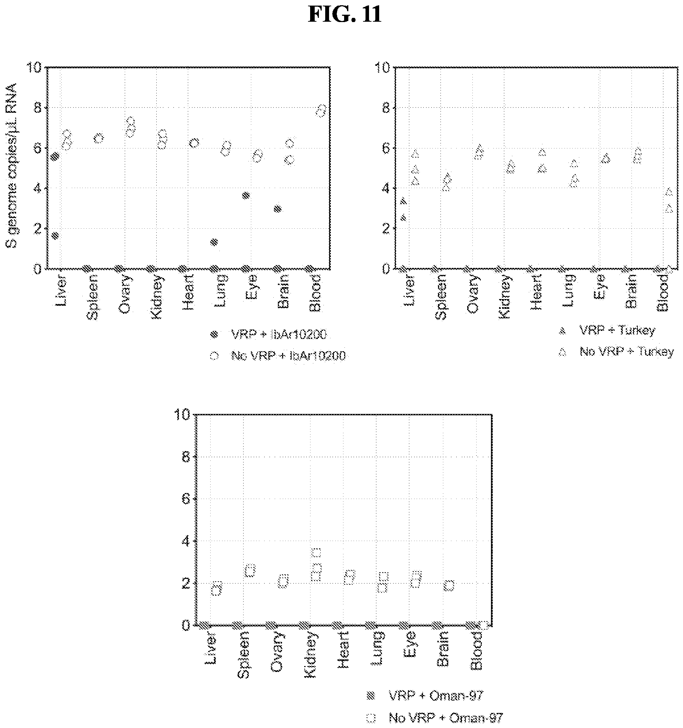

**FIG. 11** shows post-challenge RT-PCR analysis of blood and tissues. RT-PCR analyses of viral RNA (S segment) in blood and tissues from mice challenged with a target dose of 1 × 10² TCID₅₀ of indicated CCHFV strains 28 days after VRP vaccination. Tissues were collected when mice reached end-point criteria (open symbols) or at completion of the study (closed symbols; 21 days post challenge.

### SEQUENCE LISTING

The nucleic and amino acid sequences listed in the sequence listing are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand. The Sequence Listing is submitted as an ASCII text file, named Sequence_Listing.txt, created on December 13, 2019, 164 KB.
**SEQ ID NO: 1** is the nucleotide sequence of the pT7-S plasmid containing the antigenomic S segment of an exemplary CCHF VRP.
**SEQ ID NO: 2** is the nucleotide sequence of the pT7-L plasmid containing the antigenomic L segment of an exemplary CCHF VRP.
**SEQ ID NO: 3** is the nucleotide sequence of plasmid pC-N encoding an exemplary CCHF virus nucleoprotein.
**SEQ ID NO: 4** is the nucleotide sequence of plasmid pC-L encoding an exemplary CCHF virus L protein.
**SEQ ID NO: 5** is the nucleotide sequence of plasmid pC-GPC-Oman.
**SEQ ID NO: 6** is the amino acid sequence of any exemplary CCHF virus nucleoprotein.
**SEQ ID NO: 7** is the amino acid sequence of an exemplary CCHF virus L protein.
**SEQ ID NO: 8** is the amino acid sequence of modified CCHF virus L protein.
**SEQ ID NO: 9** is the amino acid sequence of the CCHF virus Oman strain GPC.
**SEQ ID NO: 10** is a codon-optimized nucleotide sequence encoding CCHF virus Oman strain GPC.
**SEQ ID NOs: 11-22** are the nucleic acid sequences of probes or primers.

### DETAILED DESCRIPTION OF SEVERAL EMBODIMENTS

It has been demonstrated that CCHF whole virus vaccines are efficacious, but face significant safety issues and manufacturing impracticalities (Dowall et al., 2017. Vaccine doi:10.1016/j.vaccine.2017.05.031; Canakoglu et al., 2015. PLoS Negl Trop Dis 9:e0003579; Mousavi-Jazi et al., 2012. Vaccine 30:6225-9; Papa et al., 2011. Scand J Infect Dis 43:225-9). In addition, subunit vaccinations against surface glycoproteins (Gn and Gc) did not protect mice from lethal CCHF virus challenges, despite the strong neutralizing activity of anti-Gc antibodies obtained (Dowall et al., 2017. Vaccine doi:10.1016/j.vaccine.2017.05.031). Recently, a DNA-based vaccine encoding the complete glycoprotein precursor (GPC) from which the structural glycoproteins (Gn and Gc) and non-structural proteins of unknown function (GP160, GP85, GP38, and NsM) are derived, or a modified vaccinia virus Ankara (MVA) vaccine have been used for protecting IFN-deficient mice (Dowall et al., 2017. Vaccine doi:10.1016/j.vaccine.2017.05.031). In addition, an adenovirus vector expressing the nucleoprotein conferred protection in mice (Zivcec M. 2013. Characterization of the Interferon αβ receptor knockout mouse model of Crimean-Congo hemorrhagic fever (CCHF) and assessment of Adenovirus based CCHF virus vaccine efficacy and correlates of protection. Ph.D. University of Manitoba, Winnipeg; Zivcec et al., 2018. PLoS Negl Trop Dis 12:e0006628). However, the DNA and MVA vaccine regimens required multiple administrations to achieve full protection, making utility limited in the context of a rapidly progressing human outbreak. Also, MVA and adenovirus vectors rely on virus delivery vehicles that themselves can be targeted by the preexisting immunity to vaccine vector. Biosafety issues have also been raised pertaining to the widespread use of MVAs (Goossens et al. 2013. Curr Gene Ther 13:413-20). To address these issues, transcriptionally competent virus-like particles (tc-VLPs) have been tried to elicit immunity only against CCHFV. However, tc-VLPs proved to provide very limited protection in preclinical models (Hinkula et al., 2017. J Virol 91).

To address these challenges, a new reverse genetics-based approach was used to generate CCHF viral replicon particles (CCHF VRPs). Unlike tc-VLPs that only include CCHF virus proteins and RNA minigenome, VRPs undergo one full round of replication closely mimicking authentic viral replication, including expression levels of CCHF virus L-protein and nucleoprotein. VRPs do not spread because they lack the M-segment which restricts *de novo* biosynthesis of GPC and spread to a single cycle of replication unless the full GPC is supplied in trans (FIG. 1). Unlike most vaccine approaches tested, VRPs do not solely rely on the expression of hypervariable GPC gene, which is unlikely on its own, to confer adequate protection against divergent strains of CCHF virus. Instead, VRPs abundantly produce the most conserved viral proteins: L-protein and nucleoprotein, and consequently build protective immunity against genetically divergent strains of CCHF virus circulating in affected countries. These methods are applicable to other nairoviruses, which do not form part of the present invention.

### I. Terms

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of the disclosure, the following explanations of specific terms are provided:
**2A Self-clearing peptides:** A class of 18-22 amino acid peptides, which can induce the cleaving of a recombination proteins in a cell based. 2A peptides are derived from the 2A region in the genome of a picornavirus. It was first identified in foot-and-mouth-disease virus (FMDV), but is also found in porcine teschovirus-1 2A (P2A), thosea asigna virus 2A (T2A), equine rhinitis A virus 2A (E2A), cytoplasmic polyhedrosis virus (BmCPV 2A) and flacherie virus (BmIFV 2A) of *B. mori.* The cleavage site is located between the last glycine of its C-terminus and the first proline of the downstream 2B protein.
**Adjuvant:** A substance or vehicle that non-specifically enhances the immune response to an antigen. Adjuvants can include a suspension of minerals (alum, aluminum hydroxide, or phosphate) on which antigen is adsorbed; or water-in-oil emulsion in which antigen solution is emulsified in mineral oil (for example, Freund's incomplete adjuvant), sometimes with the inclusion of killed mycobacteria (Freund's complete adjuvant) to further enhance antigenicity. Immunostimulatory oligonucleotides (such as those including a CpG motif) can also be used as adjuvants (for example, see U.S. Patent Nos. 6,194,388; 6,207,646; 6,214,806; 6,218,371; 6,239,116; 6,339,068; 6,406,705; and 6,429,199). Adjuvants also include biological molecules, such as costimulatory molecules. Exemplary biological adjuvants include IL-2, RANTES, GM-CSF, TNF-α, IFN-γ, G-CSF, LFA-3, CD72, B7-1, B7-2, OX-40L and 41 BBL. CCHF VRP can function as an adjuvant to enhance the immunogenicity of a heterologous vaccine.
**Administer:** As used herein, administering a composition to a subject means to give, apply or bring the composition into contact with the subject. Administration can be accomplished by any of a number of routes, such as, for example, topical, oral, subcutaneous, intramuscular, intraperitoneal, intravenous, intrathecal and intramuscular.
**Attenuated:** In the context of a live virus, the virus is attenuated if its ability to infect a cell or subject and/or its ability to produce disease is reduced (for example, eliminated) compared to a wild-type virus. Typically, an attenuated virus retains at least some capacity to elicit an immune response following administration to an immunocompetent subject. In some cases, an attenuated virus is capable of eliciting a protective immune response without causing any signs or symptoms of infection.
**Biological sample:** A sample obtained from a subject (such as a human or veterinary subject). Biological samples include, for example, fluid, cell and/or tissue samples. In some embodiments herein, the biological sample is a fluid sample. Fluid sample include, but are not limited to, serum, blood, plasma, urine, feces, saliva, cerebral spinal fluid (CSF) or other bodily fluid. Biological samples can also refer to cells or tissue samples, such as biopsy samples or tissue sections.
**Consists essentially of** and **Consists Of:** A polypeptide comprising an amino acid sequence that consists essentially of a specified amino acid sequence does not include any additional amino acid residues. However, the residues in the polypeptide can be modified to include non-peptide components, such as labels (for example, fluorescent, radioactive, or solid particle labels), sugars or lipids, and the N- or C-terminus of the polypeptide can be joined (for example, by peptide bond) to heterologous amino acids, such as a cysteine (or other) residue in the context of a linker for conjugation chemistry. A polypeptide that consists of a specified amino acid sequence does not include any additional amino acid residues, nor does it include additional biological components, such as nucleic acids lipids, sugars, nor does it include labels. However, the N- or C-terminus of the polypeptide can be joined (for example, by peptide bond) to heterologous amino acids, such as a peptide tag, or a cysteine (or other) residue in the context of a linker for conjugation chemistry.

A polypeptide that consists or consists essentially of a specified amino acid sequence can be glycosylated or have an amide modification. A polypeptide that consists of or consists essentially of a particular amino acid sequence can be linked via its N- or C-terminus to a heterologous polypeptide, such as in the case of a fusion protein containing a first polypeptide consisting or a first sequence that is linked (via peptide bond) to a heterologous polypeptide consisting of a second sequence. In another example, the N- or C-terminus of a polypeptide that consists of or consists essentially of a particular amino acid sequence can be linked to a peptide linker (via peptide bond) that is further linked to one or more additional heterologous polypeptides. In a further example, the N- or C-terminus of a polypeptide that consists of or consists essentially of a particular amino acid sequence can be linked to one or more amino acid residues that facilitate further modification or manipulation of the polypeptide.

**Crimean-Congo Hemorrhagic Fever (CCHF) virus:** A member of the genus Orthobunyavirus, family Nairoviridae (order Bunyavirales). The negative sense RNA genome is composed of three segments: Small (S), Middle (M) and Large (L). The L segment is 11-14.4 kilobases in length while the M and S segments are 4.4-6.3 and 1.7-2.1 kilobases long respectively. The L segment encodes the L protein (RNA polymerase); the M segment encodes the envelope proteins (Gc and Gn) and a variable set of non-structural proteins; and the S segment encodes the nucleocapsid protein. The CCHF L protein includes the N-terminal viral ovarian tumor (vOTU) domain (residues 1-152). The OTU domain removes ubiquitin (Ub) and Ub-like protein IFN-stimulated gene-15 (ISG15) from their protein substrates. The structure and activity of the vOTU is disclosed in Dzimianski et al., PLOS Pathogens, January 10, 2019, doi.org/10.1371/journal.ppat.1007515, incorporated herein by reference. The structure and function of the proteins of CCHF is disclosed, for example, in Capodagli et al., J. Virol. 2011;85(7):3621-30, 2014, doi: JVI.02496-10 [pii], incorporated herein by reference. The L protein is not proteolytically processed by the OTU domain. The envelope protein is initially translated as a glycoprotein precursor which is then cleaved into the mature structural glycoprotein products (Gn and Gc) and non-structural glycoproteins.

CCHF virus is not the only nairovirus that causes human disease. Dugbe virus (DUGV), Hazara (HAZV), Nairobi sheep disease virus (NSDV), Kasokero virus and Ganjam virus (GANV) all result in varying severity of febrile illness and are located in a subset of countries within the CCHF virus endemic region. Additionally, infection with NSDV and the closely related GANV in sheep negatively impacts local economies through high livestock mortality and limiting of trade with the affected areas. ERVV, found in Germany, France, Netherlands, and the Czech Republic, is increasingly implicated as the causative agent of severe headaches, known as thunderclap headaches, which result from subarachnoid hemorrhages in humans. Further information about these viruses is provided by Yadav, P. D. et al., Infect Genet Evol 11, 1111-1120, 2011; Dilcher, M. et al., Virus Genes, Aug. 7, 2012; Schwedt, T. J. et al., Lancet Neurol 5, 621-631, 2006; and Woessner, R. et al., Infection 28, 164-166, 2000, incorporated herein by reference. Further information on the CCHF virus as a model for other viruses causing hemorrhagic fever, including its structure, and biology, can be found in the following publications: Khan A, et al. Viral Hemorrhagic Fevers. Seminars in Pediatric Infectious Diseases. Philadelphia: WB Saunders Co., 1997; 8 (suppl 1):64-73; Peters C J. Viral Hemorrhagic Fevers. Viral Pathogenesis. New York: Lippincott-Raven Publishers, 1997:779-794, all incorporated herein by reference.

**Glycoprotein (GPC):** The mature CCHF virus glycoproteins, Gn and Gc (also referred to as G2 and G1), are generated by proteolytic cleavage from a precursor protein called GPC. Viral glycoproteins undergo a proteolytic processing during their biosynthesis and transport through the secretory pathway that is necessary for proper assembly and release of the infectious virus. Domains include, but are not limited to, the GP38 domain, mucin-like+GP38 domain, or an NsM, Gn, Gc receptor binding domain, discussed in more detail below and in Zivcec et al., "Molecular Insights into Crimean-Congo Hemorrhagic Fever Virus," Viruses 8 (106), 21 pages, 2016, available on-line through doi.org/10.3390/v8040106, incorporated herein by reference, domains shown in FIG. 3.

**Heterologous:** As used herein a "heterologous protein" or "heterologous virus" is a protein or virus derived from a source other than CCHF virus. In one embodiment, the heterologous ORF inserted in the S genome segment is an ORF from a different genome segment.

**Host cell:** In the context of the present disclosure, a "host cell" is a cell of use with the CCHF virus replicon system described herein. A suitable host cell is one that is capable of transfection with and expression of the plasmids of the CCHF virus replicon system. In one embodiment, the host cell is a cell expressing the CCHF virus glycoprotein (GPC). In other embodiments, a host cell can express the T7 polymerase, such as, but not limited to BSR-T7/5 cells (Buchholz et al., J. Virol. 73(1):251-259, 1999).

**Immune response:** A response of a cell of the immune system, such as a B-cell, T-cell, macrophage or polymorphonucleocyte, to a stimulus such as an antigen. An immune response can include any cell of the body involved in a host defense response, including for example, an epithelial cell that secretes an interferon or a cytokine. An immune response includes, but is not limited to, an innate immune response or inflammation. As used herein, a "protective immune response" refers to an immune response that protects a subject from infection (prevents infection or prevents the development of disease associated with infection).

**Immunize:** To render a subject protected from an infectious disease, such as by vaccination.

**Immunogen:** A compound, composition, or substance which is capable, under appropriate conditions, of stimulating an immune response, such as the production of antibodies or a T-cell response in an animal, including compositions that are injected or absorbed into an animal. Accordingly, an "immunogenic protein" is a protein capable of stimulating an immune response in a subject, such as a human or animal subject. As used herein, an "immunogenic composition" is a composition comprising an immunogen.

**Immunogenic composition:** A composition useful for stimulating or eliciting a specific immune response (or immunogenic response) in a vertebrate. The immunogenic composition includes a CCHF VRP. In some embodiments of the use in the methods, the immunogenic response is protective or provides protective immunity, in that it enables the subject to better resist infection with or disease progression from the pathogen against which the immunogenic composition is directed (*i.e.* CCHF virus). One specific example of a type of immunogenic composition is a vaccine.

**Interferon-stimulated 17 kDa protein (ISG15):** A protein that is expressed in response to interferon. ISG15 shares several properties with other ubiquitin-like molecules. Its activity is tightly regulated by specific signaling pathways that have a role in innate immunity. It also has cytokine activity. The mechanism of ISGylation is similar to that of ubiquitination.

**Isolated:** An "isolated" biological component (such as a nucleic acid, protein, virus or virus particle) has been substantially separated or purified away from other biological components (such as cell debris, or other proteins or nucleic acids). Biological components that have been "isolated" include those components purified by standard purification methods. The term also embraces recombinant nucleic acids, proteins, viruses or virus particles, as well as chemically synthesized nucleic acids or peptides.

**Nairovirus:** The family *Nairoviridae* of the order *Bunyvirales.* Nairovirus genomes are negative sense, single-stranded RNA. The complete genome is about 17,100-22,800 nucleotides long, and is divided into three segments: large, medium, and small. The large segment is about 11000-14400 nucleotides long (11-14.4 kb), and it encodes the viral polymerase. The medium segment is about 4,400-6,300 nucleotides long (4.4-6.3 kb), and it encodes the glycoproteins Gn and Gc. The small segment is about 1,700-2,100 nucleotides long (1.7-2.1 kb), and it encodes the nucleocapsid protein. The virions have a spherical shape, and range in size from about 80-120 nm in diameter. The ribonucleocapsid is filamentous. These nucleocapsids are surrounded by a single envelope that has projections made of glycoproteins protruding from its surface. In nature, nairoviruses attach to the host receptor by their Gn-Gc glycoprotein dimer. The virus is endocytosed into the host cell via a vesicle. The ribonucleocapsid segments are released into the cytoplasm, commencing transcription. Both transcription and replication occur within the cell, and newly synthesized virions are released by budding.

**ORF (open reading frame):** A series of nucleotide triplets (codons) coding for amino acids without any termination codons. These sequences are usually translatable into a peptide.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein-coding regions, in the same reading frame.

**Pharmaceutically acceptable carrier:** The pharmaceutically acceptable carriers (vehicles) useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of one or more therapeutic compounds or molecules, such as one or more CCHF virus replicon particles, and additional pharmaceutical agents.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Plasmid:** A circular nucleic acid molecule capable of autonomous replication in a host cell.

**Polypeptide:** A polymer in which the monomers are amino acid residues which are joined together through amide bonds. When the amino acids are alpha-amino acids, either the L-optical isomer or the D-optical isomer can be used. The terms "polypeptide" or "protein" as used herein are intended to encompass any amino acid sequence and include modified sequences such as glycoproteins. The term "polypeptide" is specifically intended to cover naturally occurring proteins, as well as those which are recombinantly or synthetically produced. The term "residue" or "amino acid residue" includes reference to an amino acid that is incorporated into a protein, polypeptide, or peptide.

Conservative amino acid substitutions are those substitutions that, when made, least interfere with the properties of the original protein, that is, the structure and especially the function of the protein is conserved and not significantly changed by such substitutions. Examples of conservative substitutions are shown below.

| Original Residue | Conservative Substitutions |
|---|---|
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

Conservative substitutions generally maintain (a) the structure of the polypeptide backbone in the area of the substitution, for example, as a sheet or helical conformation, (b) the charge or hydrophobicity of the molecule at the target site, or (c) the bulk of the side chain.

The substitutions which in general are expected to produce the greatest changes in protein properties will be non-conservative, for instance changes in which (a) a hydrophilic residue, for example, seryl or threonyl, is substituted for (or by) a hydrophobic residue, for example, leucyl, isoleucyl, phenylalanyl, valyl or alanyl; (b) a cysteine or proline is substituted for (or by) any other residue; (c) a residue having an electropositive side chain, for example, lysyl, arginyl, or histadyl, is substituted for (or by) an electronegative residue, for example, glutamyl or aspartyl; or (d) a residue having a bulky side chain, for example, phenylalanine, is substituted for (or by) one not having a side chain, for example, glycine.

**Preventing, treating or ameliorating a disease:** "Preventing" a disease refers to inhibiting the full development of a disease, such a CCHF. "Treating" refers to a therapeutic intervention that ameliorates a sign or symptom of a disease or pathological condition, such as CCHF, after it has begun to develop. "Ameliorating" refers to the reduction in the number or severity of signs or symptoms of a disease, such as CCHF.

**Promoter:** A promoter is an array of nucleic acid control sequences which direct transcription of a nucleic acid. A promoter includes necessary nucleic acid sequences near the start site of transcription. A promoter also optionally includes distal enhancer or repressor elements. A "constitutive promoter" is a promoter that is continuously active and is not subject to regulation by external signals or molecules. In contrast, the activity of an "inducible promoter" is regulated by an external signal or molecule (for example, a transcription factor). In some embodiments herein, the promoter is a T7 promoter (from bacteriophage T7).

**Purified:** The term "purified" does not require absolute purity; rather, it is intended as a relative term. Thus, for example, a purified peptide, protein, virus, virus particle or other active compound is one that is isolated in whole or in part from naturally associated proteins and other contaminants. In certain embodiments, the term "substantially purified" refers to a peptide, protein, virus, virus particle or other active compound that has been isolated from a cell, cell culture medium, or other crude preparation and subjected to fractionation to remove various components of the initial preparation, such as proteins, cellular debris, and other components.

**Recombinant:** A recombinant nucleic acid, protein, virus or virus particle is one that has a sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, for example, by genetic engineering techniques. In some embodiments, a recombinant CCHF virus particle is generated using the VRP system described herein.

**Reporter gene:** A reporter gene is a gene operably linked to another gene or nucleic acid sequence of interest (such as a promoter sequence). Reporter genes are used to determine whether the gene or nucleic acid of interest is expressed in a cell or has been activated in a cell. Reporter genes typically have easily identifiable characteristics, such as fluorescence, or easily assayed products, such as an enzyme. Reporter genes can also confer antibiotic resistance to a host cell or tissue. Reporter genes include, for example, GFP (or eGFP) or other fluorescence genes, luciferase, β-galactosidase and alkaline phosphatase.

**Reverse genetics:** Refers to the process of introducing mutations (such as deletions, insertions or point mutations) into the genome of an organism or virus, such as to determine the phenotypic effect of the mutation or to engineer a specific mutation or alteration.

**Sequence identity:** The similarity between amino acid or nucleic acid sequences is expressed in terms of the similarity between the sequences, otherwise referred to as sequence identity. Sequence identity is frequently measured in terms of percentage identity (or similarity or homology); the higher the percentage, the more similar the two sequences are. Homologs or variants of a given gene or protein will possess a relatively high degree of sequence identity when aligned using standard methods.

Methods of alignment of sequences for comparison are well known in the art. Various programs and alignment algorithms are described in: Smith and Waterman, Adv. Appl. Math. 2:482, 1981; Needleman and Wunsch, J. Mol. Biol. 48:443, 1970; Pearson and Lipman, Proc. Natl. Acad. Sci. U.S.A. 85:2444, 1988; Higgins and Sharp, Gene 73:237-244, 1988; Higgins and Sharp, CABIOS 5:151-153, 1989; Corpet et al., Nucleic Acids Research 16:10881-10890, 1988; Pearson and Lipman, Proc. Natl. Acad. Sci. U.S.A. 85:2444, 1988; and Altschul et al., Nature Genet. 6:119-129, 1994.

The NCBI Basic Local Alignment Search Tool (BLAST^{™}) (Altschul et al., J. Mol. Biol. 215:403-410, 1990) is available from several sources, including the National Center for Biotechnology Information (NCBI, Bethesda, MD) and on the Internet, for use in connection with the sequence analysis programs blastp, blastn, blastx, tblastn and tblastx.

**Subject:** Living multi-cellular vertebrate organisms, a category that includes both human and non-human mammals. Subjects include, but are not limited to veterinary subjects, including livestock, ostriches, rabbits, horses, donkeys, guinea pigs, hamsters, cows, goats, sheep, and non-human primates, and human subjects. Subjects also include vertebrates, such as small vertebrates and tortoises.

**Therapeutically effective amount or effective amount:** A quantity of a specified agent sufficient to achieve a desired effect in a subject being treated with that agent. For example, this may be the amount of a CCHF VRP useful for eliciting an immune response in a subject and/or for preventing or inhibiting infection by CCHF virus. Ideally, in the context of the present disclosure, a therapeutically effective amount of a CCHF VRP (or an immunogenic composition thereof) is an amount sufficient to increase resistance to, prevent, ameliorate, and/or treat infection caused by CCHF virus in a subject without causing a substantial cytotoxic effect in the subject. The effective amount of CCHF VRP useful for increasing resistance to, preventing, ameliorating, and/or treating infection in a subject will be dependent on, for example, the subject being treated, the manner of administration of the therapeutic composition and other factors.

**Transformed:** A transformed cell is a cell into which has been introduced a nucleic acid molecule by molecular biology techniques. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Ubiquitin:** A small intracellular protein that becomes conjugated to and marks proteins for destruction or for transport to particular compartments inside the cell. Ubiquitination is an enzymatic post-translational modification process in which the carboxylic acid of the terminal glycine in activated ubiquitin is catalyzed to form an amide bond to the epsilon amine of the lysine in the modified protein.

**Vaccine:** A preparation of immunogenic material capable of stimulating an immune response, administered for the prevention, amelioration, or treatment of infectious or other types of disease. The immunogenic material may include attenuated or killed microorganisms (such as attenuated viruses), or antigenic proteins, peptides or DNA derived from them. Vaccines may elicit both prophylactic (preventative) and therapeutic responses. Methods of administration vary according to the vaccine, but may include inoculation, ingestion, inhalation or other forms of administration. Inoculations can be delivered by any of a number of routes, including parenteral, such as intravenous, subcutaneous or intramuscular. Vaccines may be administered with an adjuvant to boost the immune response.

**Vector:** A nucleic acid molecule as introduced into a host cell, thereby producing a transformed host cell. A vector may include nucleic acid sequences that permit it to replicate in a host cell, such as an origin of replication (DNA sequences that participate in initiating DNA synthesis). A vector may also include one or more selectable marker genes and other genetic elements known in the art.

**Virus replicon particle (VRP):** In the context of the present disclosure, a CCHF virus replicon particle (VRP) is a viral particle that contains the CCHF nucleoprotein, glycoproteins Gc and Gn, the L protein (RNA polymerase), and packaged within the particle are the CCHF virus S (encoding the nucleoprotein) and L (encoding the L protein) genome segments. CCHF VRP do not contain the CCHF virus M segment or any nucleic acid molecule encoding functional GPC. Thus, CCHF VRP are capable of a single round of infection, including viral RNA expression and *de novo* viral protein synthesis, but cannot form new particles (due to the lack of functional Gn and Gc) and therefore cannot spread to neighboring cells. VRPs are not naturally occurring.

Unless otherwise explained, all technical and scientific terms used herein have the same meaning as commonly understood by one of ordinary skill in the art to which this disclosure belongs. As used herein, the term "comprises" means "includes." The singular terms "a," "an," and "the" include plural referents unless context clearly indicates otherwise. Similarly, the word "or" is intended to one or more than one, unless the context clearly indicates otherwise. Hence "comprising A or B" means including A, B, and both A and B. Unless otherwise indicated, "about" indicates within 5%. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of the present disclosure, suitable methods and materials are described below. In case of conflict, the present specification, including explanations of terms, will control. In addition, the materials, methods, and examples are illustrative only and not intended to be limiting.

### II. CCHF VRP and Overview of Several Embodiments

Disclosed herein is the development and characterization of Crimean-Congo hemorrhagic fever (CCHF) viral replicon particles (VRP). These VRP are replication-competent but non-spreading virus particles. It is disclosed herein that these VRP are a safe and rapidly efficacious immunogenic composition (such as a vaccine) for protection against CCHF virus infection. In particular embodiments, the VRP contain one or more mutations in the viral ovarian tumor domain protease. These VRP do not carry the genes for structural glycoproteins and are therefore unable to produce new particles from infected cells, preventing spread within the immunized host and eliminating the risk of vaccine-induced pathogenicity. Thus, the VRP do not encode the full length viral glycoprotein (GPC). The CCHF VRP a) does not contain a CCHF virus M segment or b) encodes a domain of GPC but not a full-length GPC. In some non-limiting examples, nucleic acid molecules encoding one or more domains of the GPC, but not a full-length GPC are included in the S segment, such as a modified P2A S segment.

In specific non-limiting examples, the domain of the GPC is a mucin-like domain, GP38 domain, mucin-like+GP38 domain, or an NsM, Gn, Gc receptor binding domain. Similar VRP can be produced for other nairoviruses. Domains of the GPC are known in the art. For example, in CCHF GPC, suitable domains include: a) mucin-like domain (28-250), b) GP38 domain (250-522), c) mucin-like+GP38 domains (28-522), d) domains NsM (825-1003), e) Gn (523-824), f) Gc receptor binding domains (1223-1423). See also Xiao et al., Biochem. Biophys. Res. Comm. 411: 253-258, 2011, incorporated herein by reference. SEQ ID NO: 9 provides a full-length GPC, with all domains. Gn together with Gc mediates CCHFV cell entry. The Gc receptor binding domain binds the cell surface nucleolin. In some embodiments, nucleic acid molecule(s) encoding the one or more domains are inserted in the S segment, such as the modified P2A segment, see FIG. 2.

The data disclosed herein demonstrate that CCHF VRP immunization is both safe and efficacious against virulent CCHF virus challenge in a relevant animal model. Immunization with non-replicating CCHF VRP resulted in a significant increase survival following CCHF virus challenge. The animals also developed a strong immune response.

The CCHF virus shares the same replication pathway with other nairoviruses such as human disease-causing Issyk-kul, Kasokero virus, Dugbe, and Erve nairoviruses, as well as economically important Nairobi Sheep Disease (including the Ganjam Variant). Thus, similar VRPs can be produced and used to provide protection for humans and animals across a broad range of nairoviruses. Other nairoviruses include, but are not limited to: Dugbe orthonairovirus, Erve orthonairovirus, Taggert orthonairovirus, Hazara orthonairovirus, Kupe orthonairovirus, Farallon orthonairovirus, Nairobi Sheep disease orthonairovirus, Nairobi Sheep disease orthonairovirus strain Ganjam, Issyk-kul orthonairovirus, Qalyub orthonairovirus, Leopard Hills orthonairovirus, Thiafora orthonairovirus, Sapphire II orthonairovirus, Huangpi orthonairovirus, Tillamook orthonairovirus and Kasokero orthonairovirus. Similar VRP can be produced from these nairoviruses, and these VRP can be used in methods for inducing an immune response. The discussion below refers to CCHF VRP, but is applicable to any nairovirus. VRP can be produced from Issyk-kul, Dugbe, and Erve nairoviruses, Nairobi Sheep Disease (including the Ganjam Variant), Dugbe orthonairovirus, Erve orthonairovirus, Taggert orthonairovirus, Hazara orthonairovirus, Kupe orthonairovirus, Farallon orthonairovirus, Nairobi Sheep disease orthonairovirus, Nairobi Sheep disease orthonairovirus strain Ganjam, Issyk-kul orthonairovirus, Qalyub orthonairovirus, Leopard Hills orthonairovirus, Thiafora orthonairovirus, Sapphire II orthonairovirus, Huangpi orthonairovirus, Tillamook orthonairovirus and Kasokero orthonairovirus. These VRP are of use for inducing an immune response to the respective nairovirus.

The CCHF VRP disclosed herein include a virus particle that contains a CCHF virus L genome segment and a CCHF virus S genome segment, but does not contain a CCHF virus M genome segment, or any nucleic acid that encodes GPC. However, in some embodiments, the S genome segment encodes a portion of Gn and/or Gc, such as a Mucin-like, GP38, Mucin+GP38, Gn, Nsm, Gc receptor binding domains (see Zivcec et al., "Molecular Insights into Crimean-Congo Hemorrhagic Fever Virus," Viruses 8 (106), 21 pages, 2016, available on-line through doi.org/10.3390/v8040106, incorporated herein by reference, domains shown in FIG. 3). For example, when the GPC is synthesized in the endoplasmic reticulum, the mucin domain is amino acid position 1-247, GP38 is amino acid position 248 to 519, Gn is amino acid position 520 to 843, the Nsm domain is amino acid position 844 to 1040, and the Gc domain is amino acid position 1041 to 1684. The protein components of the VRP include CCHF virus Gn and Gc glycoproteins, L protein and nucleoprotein.

Provided herein is a CCHF VRP, comprising (i) CCHF virus Gn and Gc glycoproteins; (ii) CCHF virus L protein; (iii) CCHF virus nucleoprotein; (iv) a CCHF virus L genome segment; and (v) a CCHF virus S genome segment, wherein the VRP does not contain an CCHF virus M segment or any nucleic acid molecule encoding functional/full-length CCHF virus Gn and Gc glycoproteins.

The CCHF virus from which the CCHF virus genome segments and proteins are derived can be any strain of CCHF virus, or can be from more than one strain. For example, the genome segments can be derived from a first strain, and one or more proteins can be derived from a second strain. In some embodiments, the CCHF virus is African strain IbAr10200. In other embodiments, the CCHF virus is CCHF virus Asia (Oman1998) strain or Europe (Turkey2004) strain. In yet other embodiments, the CCHF orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, or orthonairovirus strain Iran Kerman/22.

In some embodiments, the L genome segment includes one or more mutations in a viral ovarian tumor domain protease (vOTU) domain of the L genome segment. In some examples, the one or more mutations disrupt vOTU deubiquitinase activity and/or ISG15 activity. In some embodiments, the mutation can reduce the vOTU activity bu at least about 20%, 30%, 40%, 50%, 60%, 70%, 80%, 90%, 95%, 96%, 98% or 99%. In some embodiments, the mutation can increase the ISG15 activity by at least about 20%, 30%, 40%, 50%, 60%, 70%, 80%, 90%, 100%, 200% or 300%. In some non-limiting examples, the one or more mutations comprise a Q16R mutation (with reference to SEQ ID NO: 8), and wherein vOTU deubiquitinase activity is disrupted. In other non-limiting examples, the one or more mutations comprise or further comprise at least one of I13R/E/K, V18I, C40A/S/R, P77D/T, T120L, E128V and A129R/G (with reference to SEQ ID NO: 8).

In some embodiments, the amino acid sequence of the L protein is at least 80%, at least 85%, at least 90%, at least 95%, at least 96%, at least 97%, at least 98% or at least 99% identical to SEQ ID NO: 7 or SEQ ID NO: 8. In some examples, the amino acid sequence of the L protein comprises or consists of SEQ ID NO: 7 or SEQ ID NO: 8.

In some embodiments, the amino acid sequence of GPC is at least 80%, at least 85%, at least 90%, at least 95%, at least 96%, at least 97%, at least 98% or at least 99% identical to SEQ ID NO: 9. In some examples, the amino acid sequence of GPC comprises or consists of SEQ ID NO: 9.

In some embodiments, the amino acid sequence of the nucleoprotein is at least 80%, at least 85%, at least 90%, at least 95%, at least 96%, at least 97%, at least 98% or at least 99% identical to SEQ ID NO: 6. In some examples, the amino acid sequence of the nucleoprotein comprises or consists of SEQ ID NO: 6.

In some embodiments, the L genome segment comprises a nucleotide sequence at least 80%, at least 85%, at least 90%, at least 95%, at least 96%, at least 97%, at least 98% or at least 99% identical to nucleotides 2706-14865 of SEQ ID NO: 2. In some examples, the nucleotide sequence of the L genome segment comprises or consists of nucleotides 2706-14865 of SEQ ID NO: 2.

In some embodiments, the S genome segment comprises a nucleotide sequence at least 80%, at least 85%, at least 90%, at least 95%, at least 96%, at least 97%, at least 98% or at least 99% identical to nucleotides 2706-4377 of SEQ ID NO: 1. In some examples, the nucleotide sequence of the S genome segment comprises or consists of nucleotides 2706-4377 of SEQ ID NO: 1.

In some embodiments, the CCHF virus S and L segments are at least 80%, at least 85%, at least 90%, at least 95%, at least 96%, at least 97%, at least 98% or at least 99% identical to an S or L segment disclosed in U.S. Patent No. 9,474,796.

In some embodiments, the plasmid containing an antigenomic copy of the S segment and the plasmid containing an antigenomic copy of the L segment further comprise a T7 promoter and a hepatitis delta virus ribozyme. In some embodiments, the host cells express T7 polymerase.

In some embodiments, the CCHF VRP S genome segment comprises a CCHF virus nucleoprotein open reading frame (ORF) and a heterologous ORF. In some examples, the heterologous ORF encodes a portion of a CCHF virus GPC (but not functional or full-length GPC). In some examples, the heterologous ORF encodes a protein from an infectious organism, such as a heterologous virus (a virus other than CCHF virus). The heterologous ORF can encode, for example, an immunogenic protein from a heterologous virus (a virus other than CCHF virus), or from any other infectious organism against which an immune response is desired. In some cases, the heterologous protein is a vaccine or a component of a vaccine for an infectious organism, such as a bacterial or viral organism. Non-limiting examples are antigens are Rift Valley fever virus antigens, such as the nucleoprotein (NP) or the Gc, Ebola virus antigens, such as the glycoprotein (GP), Marburg virus antigens, such as the GP, or Nipah virus G antigen.

In other examples, the heterologous ORF encodes a reporter. Any gene that produces a protein with a functional readout can be used as the reporter gene. Reporter genes include, but are not limited to genes encoding fluorescent proteins, antibiotic resistance or enzymes (*e.g.*, β-galactosidase or alkaline phosphatase). In some examples, the reporter gene is a GFP, such as enhanced GFP, or ZsGreen.

In some embodiments, the nucleoprotein ORF and the heterologous ORF are in-frame and are separated by the coding sequence for a self-cleaving 2A peptide. In some examples, the 2A peptide comprises a porcine teschovirus-1 (PTV1) 2A (P2A) peptide, a foot and mouth disease virus (FMDV) 2A (F2A) peptide, an equine rhinitis A virus (ERAV) 2A (E2A) peptide or a Thosea asigna virus (TaV) 2A (T2A) peptide (see FIG. 2).

Methods are provided for producing a CCHF VRP comprising (i) transfecting a host cell with a plasmid containing an antigenomic copy of a CCHF virus L segment; a plasmid containing an antigenomic copy of a CCHF virus S segment; a plasmid encoding CCHF virus GPC; a plasmid encoding a CCHF virus nucleoprotein; and a plasmid encoding a CCHF virus L protein; and (ii) culturing the cells for a period of time sufficient to produce CCHF VRP. The CCHF VRP includes a CCHF virus L genome segment, a CCHF virus S genome segment, Gn and Gc glycoproteins, L protein and nucleoprotein. Optionally, a plasmid encoding the bacteriophage T7 RNA polymerase is utilized, when a T7 promoter is included in the constructs. However, other promoter systems are also of use.

In some embodiments, the method further includes collecting the CCHF VRP from the cell culture supernatant. In some examples, the method further includes isolating or purifying the CCHF VRP from the cell culture supernatant.

In some embodiments, the method further includes amplifying the CCHF VRP in a susceptible cell line expressing CCHF virus GPC in trans. For example, the isolated or purified CCHF VRP can be used to infect a cell line that has been transformed with a plasmid expressing the CCHF virus GPC, thereby enabling amplification of the VRP.

The CCHF virus genome segments and proteins used with the disclosed methods can be derived from any strain of CCHF virus, or can be from more than one strain of CCHF virus. For example, the genome segments can be derived from a first strain, and one or more proteins can be derived from a second strain. In some embodiments, the CCHF virus is African strain IbAr10200. In other embodiments, the CCHF virus is CCHF virus Asia (Oman1998) strain or Europe (Turkey2004) strain. In yet other embodiments, the CCHF orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, or orthonairovirus strain Iran Kerman/22.

In some embodiments of the methods, the L genome segment includes one or more mutations in a vOTU domain of the L genome segment. In some examples, the one or more mutations disrupt vOTU deubiquitinase activity and/or ISG15 activity. In some non-limiting examples, the one or more mutations comprise a Q16R mutation (SEQ ID NO: 8), and wherein vOTU deubiquitinase activity is disrupted. In other non-limiting examples, the one or more mutations comprise or further comprise at least one of I13R/E/K, V18I, C40A/S/R, P77D/T, T120L, E128V and A129R/G (SEQ ID NO: 8).

In some embodiments of the methods, the plasmid comprising an antigenomic copy of the S segment and the plasmid comprising an antigenomic copy of the L segment further include a T7 promoter operably linked at the 5' end of the antigenomic copy and a hepatitis delta virus ribozyme at the 3' end of the antigenomic copy.

In some embodiments, the plasmid comprising an antigenomic copy of the L segment comprises a nucleotide sequence that is at least 85%, at least 90%, at least 95%, at least 96%, at least 97%, at least 98% or at least 99% identical to SEQ ID NO: 2. In some examples, the nucleotide sequence of the plasmid comprising an antigenomic copy of the L segment comprises or consists of SEQ ID NO: 2.

In some embodiments, the plasmid comprising an antigenomic copy of the S segment comprises a nucleotide sequence that is at least 85%, at least 90%, at least 95%, at least 96%, at least 97%, at least 98% or at least 99% identical to SEQ ID NO: 1. In some examples, the nucleotide sequence of the plasmid comprising an antigenomic copy of the S segment comprises or consists of the nucleotide sequence of SEQ ID NO: 1.

In some embodiments, the nucleotide sequence of the plasmid encoding the CCHF virus GPC is at least 85%, at least 90%, at least 95%, at least 96%, at least 97%, at least 98% or at least 99% identical to SEQ ID NO: 5. In some examples, the nucleotide sequence of the plasmid encoding the GPC comprises or consists of the nucleotide sequence of SEQ ID NO: 5.

In some embodiments, the nucleotide sequence of the plasmid encoding the CCHF virus nucleoprotein comprises a nucleotide sequence that is at least 85%, at least 90%, at least 95%, at least 96%, at least 97%, at least 98% or at least 99% identical to SEQ ID NO: 3. In some examples, the nucleotide sequence of the plasmid encoding the nucleoprotein comprises or consists of the nucleotide sequence of SEQ ID NO: 3.

In some embodiments, the nucleotide sequence of the plasmid encoding the CCHF virus L protein comprises a nucleotide sequence that is at least 85%, at least 90%, at least 95%, at least 96%, at least 97%, at least 98% or at least 99% identical to SEQ ID NO: 4. In some examples, the nucleotide sequence of the plasmid encoding the L protein comprises or consists of the nucleotide sequence of SEQ ID NO: 4.

Further provided here are immunogenic compositions that include a CCHF VRP disclosed herein, and a pharmaceutically acceptable carrier. In some embodiments, the immunogenic composition further includes an adjuvant.

Also provided is the immunogenic composition for use in a method of eliciting an immune response against CCHF virus in a subject by administering to the subject an effective amount of the immunogenic composition disclosed herein.

Further provided is the immunogenic composition for use in a method of immunizing a subject against CCHF virus infection by administering to the subject an effective amount of the immunogenic composition disclosed herein. In specific non-limiting examples, the subject is a human. In other non-limiting examples, the immunogenic composition for use in the method produces a protective immune response.

Also provided is the immunogenic composition for use in a method of eliciting an immune response against a CCHF viral protein in a subject is also provided. In some embodiments, the use in the method includes administering to the subject an immunogenic composition disclosed herein, wherein the viral protein is a CCHF virus nucleoprotein or a CCH virus L protein.

In some embodiments of the immunogenic composition for use in the methods, the subject is human.

In some embodiments of the immunogenic composition for use in the methods, only one dose of the immunogenic composition is administered to the subject.

In some embodiments of the immunogenic composition for use in the methods, the immunogenic composition is administered intravenously, intramuscularly or subcutaneously.

### III. Immunogenic Compositions and Administration of CCHF VRP

Immunogenic compositions comprising the CCHF VRP as defined in the claims for use in the methods defined in the claims, can be administered to a subject by any of the routes normally used for introducing virus or virus particles into a subject. Methods of administration include, but are not limited to, intradermal, intramuscular, intraperitoneal, parenteral, intravenous, subcutaneous, vaginal, rectal, intranasal, inhalation or oral. Parenteral administration, such as subcutaneous, intravenous or intramuscular administration, is generally achieved by injection. Injectables can be prepared in conventional forms, either as liquid solutions or suspensions, solid forms suitable for solution or suspension in liquid prior to injection, or as emulsions. Injection solutions and suspensions can be prepared from sterile powders, granules, and tablets of the kind previously described. Administration can be systemic or local.

Immunogenic compositions are administered in any suitable manner, such as with pharmaceutically acceptable carriers. Pharmaceutically acceptable carriers are determined in part by the particular composition being administered, as well as by the particular method used to administer the composition. Accordingly, there is a wide variety of suitable formulations of pharmaceutical compositions of the present disclosure.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Some of the compositions may potentially be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines. Optionally, the composition can include an adjuvant.

Administration can be accomplished by single or multiple doses. In some embodiments of the use in the methods, only a single dose of the composition is administered to the subject. The dose administered to a subject in the context of the present disclosure should be sufficient to induce a beneficial immune response in a subject over time, or to inhibit or prevent a CCHF virus infection. The dose required will vary from subject to subject depending on the species, age, weight and general condition of the subject, the severity of the infection being treated, the particular immunogenic composition being used and its mode of administration. An appropriate dose can be determined by one of ordinary skill in the art using only routine experimentation. A prime boost strategy can be utilized. In one embodiment of the use in the methods, only a single administration is used.

Provided herein are pharmaceutical compositions (also referred to as immunogenic compositions) which include a therapeutically effective amount of the CCHF VRP, alone or in combination with a pharmaceutically acceptable carrier. Pharmaceutically acceptable carriers include, but are not limited to, saline, buffered saline, dextrose, water, glycerol, ethanol, and combinations thereof. The carrier and composition can be sterile, and the formulation suits the mode of administration. The composition can also contain minor amounts of wetting or emulsifying agents, or pH buffering agents. The composition can be a liquid solution, suspension, emulsion, tablet, pill, capsule, sustained release formulation, or powder. The composition can be formulated as a suppository, with traditional binders and carriers such as triglycerides. Oral formulations can include standard carriers such as pharmaceutical grades of mannitol, lactose, starch, magnesium stearate, sodium saccharine, cellulose, and magnesium carbonate. Any of the common pharmaceutical carriers, such as sterile saline solution or sesame oil, can be used. The medium can also contain conventional pharmaceutical adjunct materials such as, for example, pharmaceutically acceptable salts to adjust the osmotic pressure, buffers, preservatives and the like. Other media that can be used with the compositions and methods provided herein are normal saline and sesame oil.

The CCHF VRP disclosed herein, and immunogenic compositions including these VRP can be administered alone or in combination with other therapeutic agents to enhance antigenicity. For example, the VRP can be administered with an adjuvant, such as Freund incomplete adjuvant or Freund's complete adjuvant. These adjuvants can be included in the immunogenic compositions. The VRP of the invention is a CCHF VRP as defined in the claims.

Optionally, one or more cytokines, such as IL-2, IL-6, IL-12, RANTES, GM-CSF, TNF-α, or IFN-γ, one or more growth factors, such as GM-CSF or G-CSF; one or more molecules such as OX-40L or 41 BBL, or combinations of these molecules, can be used as biological adjuvants (see, for example, Salgaller et al., 1998, J. Surg. Oncol. 68(2): 122-38; Lotze et al., 2000, Cancer J. Sci. Am. 6(Suppl 1): S61-6; Cao et al., 1998, Stem Cells 16(Suppl 1):251-60; Kuiper et al., 2000, Adv. Exp. Med. Biol. 465:381-90). These molecules can be administered systemically (or locally) to the host, either in the same compositions, simultaneously in a different composition, or sequentially.

### VI. Use of VRP and Immunogenic Compositions Thereof

The immunogenic compositions comprising CCHF VRP, can be used, for example, to elicit an immune response in a subject or to immunize a subject against CCHF. The immunogenic compositions disclosed herein are used as vaccines to prevent or inhibit infection by CCHF virus or CCHF virus infection, in humans or other mammals. Suitable subjects include, but are not limited to veterinary subjects, including livestock, buffalos, camels, chickens, ducks, dogs, ostriches, rabbits, horses, donkeys, guinea pigs, hamsters, cows, goats, sheep, non-human primates, and human subjects. Subjects also include small vertebrates and tortoises. In a specific non-limiting example, the subject is a human. The disclosed immunogenic compositions can be used to prevent or treat a CCHF virus infection. The disclosed use in the methods can reduce a symptom of a CCHF infection and/or reduce viral titer.

The CCHF VRP, and immunogenic compositions disclosed herein can be targeted towards veterinary medical use, and thus indirectly prevent human disease, such as CCHF disease. However, the candidate vaccines can also provide effective therapeutic and prophylactic protection for humans, such as those in high risk occupational settings, or in recognized risk groups following natural or intentional introduction of nairovirus, such as CCHF virus, into previously unaffected areas.

The invention provides the immunogenic composition as defined in the claims for use in a method of eliciting an immune response a CCHF virus, or CCHF viral protein, in a subject. In some embodiments, the use in the method includes administering to the subject an effective amount of CCH VRP or an immunogenic composition disclosed herein. Further provided is the immunogenic composition as defined in the claims for use in a method of immunizing a subject against CCHF virus infection by administering to the subject an effective amount of the immunogenic composition disclosed herein. Other immunogenic compositions including nairovirus VRPs can be similarly produced and used to immunize a subject against the respective nairovirus, which does not form part of the invention. In some embodiments, the use in the method elicits an immune response against more than one strain of CCHFV. The method can induce an immune response against multiple strains of CCHFV. The method can elicit an immune response to one or more of orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, and orthonairovirus strain Iran Kerman/22. The method can elicit an immune response to an African-clade, Europe-clade, and/or Asia clade CCHFV. The method can elicit an immune response to Turkey and/or Oman strains of CCHFV.

In some embodiments, the subject is a human. In other embodiments, the subject is a veterinary subject.

In some embodiments of the use in the methods, the immunogenic composition is administered in a single dose. In another embodiment of the use in the methods, the immunogenic composition is administered in multiple doses, such as two, three or four doses. When administered in multiple doses, the time period between doses can vary. In some cases, the time period is days, weeks or months. The CCHF VRP, or immunogenic composition can be administered using any suitable route of administration. In some embodiments of the use in the methods, the CCHF VRP, or immunogenic composition is administered intravenously, intramuscularly or subcutaneously. The administration can include a prime and one, or more than one, boost. In some embodiments of the use in the methods, a single dose elicits an immune response against multiple strains of CCHFV. In other embodiments of the use in the methods, a single dose elicits an immune response to an African-clade, Europe-clade, and/or Asia clade CCHFV. In more embodiments of the use in the methods, a single dose elicits an immune response to Turkey and/or Oman strains of CCHFV. In more embodiments of the use in the methods, a single dose elicits elicit an immune response to CCHF orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, or orthonairovirus strain Iran Kerman/22. In other embodiments of the use in the methods, a single dose elicits an immune response to at least 1, 2, 3, 4, 5, 6, 7, 8, 9, 10, 11 or all of CCHF orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, or orthonairovirus strain Iran Kerman/22.

A suitable dose of a CCHF VRP, (or immunogenic composition comprising the CCHF VRP) can be selected by a skilled practitioner. In some embodiments of the use in the methods, the VRP is administered at a dose of about 10² to about 10⁶ TCID₅₀. In some examples, the VRP is administered at a dose of about 10³ to about 10⁵ TCID₅₀. In particular examples, the VRP is administered at a dose of about 10², about 10³, about 10⁴, about 10⁵ or about 10⁶ TCID₅₀. In specific non-limiting examples, a CCHF VRP is utilized.

Further provided is a method of enhancing an immune response against a heterologous immunogenic protein, such as an immunogenic viral protein, in a subject by administering the immunogenic protein to the subject and further administering a nairovirus VRP, such as a CCHF VRP which does not form part of the invention. It is disclosed herein that nairovirus VRP, such as CCHF VRP, can also be used as vaccine vectors to elicit an immune response against a heterologous protein. Thus, in some examples, the S segment includes an ORF encoding a heterologous protein. The heterologous protein can be an immunogenic protein from a heterologous virus (any virus other than the nairovirus, such as the CCHF virus), or from any other infectious organism against which an immune response is desired. In some cases, the heterologous protein is a vaccine or a component of a vaccine for an infectious organism, such as a bacterial or viral organism. In some examples, the heterologous ORF encodes a portion of CCHF virus GPC. In yet other examples, the heterologous ORF encodes a reporter protein, such as a fluorescent protein.

Also provided is the use of a nairovirus VRP, such as a CCHF VRP as an adjuvant to enhance the immunogenicity of a heterologous protein, such as a heterologous vaccine, which does not form part of the claimed invention. Thus, in some aspects of the disclosure, the immunogenic compositions provided herein further include an immunogenic protein from a heterologous virus (any virus other than the nairovirus, such as the CCHF virus), or any other infectious microorganism. Thus, provided is a method of eliciting an immune response against a heterologous protein in a subject, by administering to the subject a nairovirus VRP, such as a CCHF VRP in which the S segment includes an ORF encoding the heterologous protein, which does not form part of the claimed invention.

Without being bound by theory, the nairovirus VRP, such as the CCHF disclosed herein, have several distinct advantages over prior CCHF virus vaccines - they exhibit rapid and robust efficacy, enhanced safety, are incapable of reverting to a virulent virus, and they induce an immune response with a single administration. Furthermore, they increase survival in an animal model. In addition, the disclosed VRP can be used in induce an immune response to one or more heterologous strains. In other embodiments of the use in the methods, a single dose elicits an immune response to an African-clade, Europe-clade, and/or Asia clade CCHFV, and can include gene segments from viruses in one or more of these clades. In some embodiments" the VRP includes gene segments from CCHF orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, or orthonairovirus strain Iran Kerman/22, and administration produces an immune response to more that one of CCHF orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, or orthonairovirus strain Iran Kerman/22. Thus, the VRP can be cross-protective.

In some embodiments of the use in the methods, a single administration of the composition may be sufficient to raise a protective immune response. Multiple administrations can also be used. Desirable outcomes include induction or enhancement of a specific antibody response measured by a suitable test, such as enzyme-linked immunosorbant assay (ELISA) using viral antigens, or a virus neutralization assay. Desirable outcomes also include the development of a T cell response, such as induction of an antigen-specific CD4 and/or CD8+ T cell response.

For purposes of treatment or eradication of an ongoing infection, single or multiple administrations can be used. In one non-limiting example, only a single administration is used. Desirable outcomes also include the development of a T cell response, such as induction of an antigen-specific CD4 and/or CD8+ T cell response. Clinical benefit can be measured as a reduction in the titer of virus or infectious particles in blood or in a tissue biopsy, or a limitation in the progression of necrosis, pain, wasting, or other signs of the disease. Desirable outcomes also include the product of an immune response to more than one strain of CCHF virus. In some embodiments of the use in the methods, immune response to an African-clade, Europe-clade, and/or Asia clade CCHFV is produced. In some embodiments of the use in the methods, an immune response is produced to one or more of CCHF orthonairovirus strain 10200, orthonairovirus strain Oman1997, orthonairovirus strain Kosovo Hoti, orthonairovirus strain AP-92, orthonairovirus strain Senegal ArD15786, orthonairovirus strain Mauritania 39554, orthonairovirus strain Turkey-Kelkit06, orthonairovirus strain Turkey200310849, orthonairovirus strain India NIV11703, orthonairovirus strain Uganda UG3010, orthonairovirus strain Sudan Al Fulah-2008, or orthonairovirus strain Iran Kerman/22.

In some embodiments of the use in the methods, the subject is further administered a second anti-viral agent. Exemplary anti-viral agents include, but are not limited to, ribavirin, an IgG, or a monoclonal antibody. Additional agents include, but are not limited to, favipiravir, site-1 protease inhibitor, CCHF ovarian tumor domain inhibitor, 2'-deoxy-2'-fluorocytidine, and mycophenolic acid.

The following examples are provided to illustrate certain particular features and/or embodiments. These examples should not be construed to limit the disclosure to the particular features or embodiments described. Any examples falling outside the scope of the claims are provide for comparative purposes only.

### EXAMPLES

### Example 1: Development and characterization of CCHF virus replicon particles (VRPs)

This example describes the use of a new reverse genetics-based approach to generate CCHF viral replicon particles (CCHF VRPs). Unlike transcriptionally competent VLPs (tc-VLPs), which only include CCHF viral proteins, VRPs undergo one full round of replication, closely mimicking authentic viral replication, including expression levels of CCHF virus L protein and nucleoprotein. VRPs do not spread because they lack the GPC-encoding M-segment. VRPs undergo a single cycle of replication, unless the full GPC is supplied *in trans* (FIG. 1, bottom). Unlike most vaccine approaches tested, VRPs do not solely rely on the expression of the hypervariable GPC gene, which is unlikely on its own to confer adequate protection against divergent strains of CCHF virus. Instead, VRPs abundantly produce the most conserved viral proteins, L protein and nucleoprotein, and consequently are capable of eliciting protective immunity against genetically divergent strains of CCHF virus circulating in affected countries.

### Plasmids

The pT7 vector was previously described (Albarino et al., J Virol 83(11):5606-5614, 2009); it contains a BsmB1 cloning site located between a T7 promoter and a hepatitis D ribozyme T7 polymerase terminator motif. The S, M, and L cDNAs were cloned into the BsmB1-digested pT7 vector in viral complementary orientation relative to the T7 promoter. Primary T7 transcripts derived from pT7-S (GenBank^{™} Accession No. KJ648914; SEQ ID NO: 1), pT7-M (GenBank^{™} Accession No. KJ648915), and pT7-L (GenBank^{™} Accession No. KJ648913; SEQ ID NO: 2) contain an artificial G at the 5' ends, but this nucleotide is rapidly lost upon replication (Bergeron et al., J Virol 84:216-226, 2010). pT7-S was modified by inserting the ZsGreen1 (ZsG) coding sequence fused to the P2A sequence upstream of the NP coding region (pT7-P2A-S ZsG). CCHF nucleoprotein, L protein and glycoprotein precursor (GPC) were cloned into mammalian expression vector pCAGGS (pC). pC-N (GenBank^{™} Accession No. KJ648912; SEQ ID NO: 3) contains strain IbAr10200 nucleoprotein, pC-L opti (GenBank^{™} Accession No. KJ648910; SEQ ID NO: 4) was obtained by optimizing the codons of strain IbAr10200 L ORF for maximal expression in human cells (GeneArt, Ratisbonne, Germany). pC-L opti was tagged with an N-terminal V5 epitope. pC-T7 was obtained by cloning human codon-optimized bacteriophage T7 RNA Pol (Genscript, Piscataway, NJ, USA). pC-GPC-Oman was obtained by cloning a codon-optimized sequence of the GPC of Oman 1998 strain (SEQ ID NO: 5).

### Rescue and propagation of CCHF VRP vaccine

Six-well plates were seeded with 3.5 × 10⁵ Huh7 cells/well 1 day prior to transfection in 3 mL of DMEM supplemented with 1% non-essential amino acids, 1 mM sodium pyruvate, and 10% FBS. 16-24 hours later, cells were transfected with pT7-S-IbAr10200 (1 µg) or pT7-S P2A zsGreen (1 µg) together with pT7-L-IbAr10200 (1 µg), pC-L-IbAr10200 (0.33 µg), pC-N-IbAr12000 (0.66 µg) and pC-GPC-Oman (1 µg) and pC-T7 (1 µg), combined with 12.5 µL of Mirus LT1 transfection reagent (Mirus Bio, Madison, WI, USA) in 250 µL of OPTI-MEM (Life Technologies, Grand Island, NY, USA). Transfected cell supernatants containing VRPs were harvested 4-5 days post-transfection. To obtain high titers, VRP were further propagated in Huh7 cells transfected with pC-GPC-Oman using Mirus LT1 reagent. Stocks of VRPs were titrated by immunofluorescence with rabbit anti-CCHFV antibody using 50% tissue culture infective dose (TCID50) using the Reed-Muench method.

### Mice immunization and challenge

Female IFNAR KO mice (MMRRC/The Jackson Laboratory) were housed in HEPA-filtered micro-isolator cage systems. Ten mice per group were vaccinated subcutaneously at 8 weeks of age with either a high (4.39×10⁵) or low (4.64×10³) VRP dose. Four mice were mock-vaccinated with DMEM only. Twenty-eight days post vaccination, blood samples were collected via the mandibular vein. Prior to challenge, one mouse was removed from the 'low VRP dose' group due to unrelated health issues. Thirty-two days post vaccination, animals were challenged with a lethal dose of CCHFV (100 TCID₅₀). Animals were monitored daily for signs of clinical illness. Animals were humanely euthanized once clinical illness scores (including, but not limited to, piloerection, weight loss >25%, changes in mentation, ataxia, dehydration, or dyspnea) indicated that the animal was in the terminal stages of disease, or at the completion of study (18 days post challenge).

### Results

It was demonstrated that CCHF VRPs can be produced and they undergo only one round of replication as they lack the M segment, but can be amplified by expressing GPC *in trans* (FIG. 1). CCHF VRPs represent a valuable tool to study L protein function in a natural context without the confounding effects associated with virus spread. Prior infection with CCHF virus is believed to confer long-term protection, as no cases of CCHF virus reinfection case have been reported. To determine the baseline levels of protection conferred by VRP vaccination, interferon (IFN)-R KO mice were immunized with either a low dose (4.64 ×10³ TCID₅₀) or a high dose (4.39 × 10⁵ TCID₅₀) of VRPs using the wild-type L and S of CCHF virus African strain IbAr10200. Prior to challenge, levels of Gc and NP antibody were determined by ELISA. All mice immunized with a high dose of VRPs developed Gc and NP antibodies versus 90% and 50% of the animals receiving a low dose developed Gc and NP antibodies. Gc and antibody levels were found higher in group receiving a high dose (FIG. 6). None of the vaccinated animals showed any weight loss or sign of disease prior to uniformly lethal CCHF virus challenge 32 days post vaccination. All mock-treated mice succumbed within 5 days after challenge. None of the vaccinated mice with a high dose of VRPs showed weight lost or sign of disease (n=10) after challenge. In comparison, 7/9 mice receiving a low dose survived and 2/9 succumbed 3-4 days later than the mock control group. These results demonstrated that a single vaccination can provide 100% protection in a very susceptible animal model.

VRP efficacy was improved by altering the vOTU activity of VRPs and introducing portions of the GPC gene in the VRP S-segment (FIG. 2). To demonstrate that modifying vOTU increases the innate immune response in the context of CCHF VRPs infection, VRPs were produced with the Q16R mutation that disrupts vOTU deubiquitinase activity. Infection of THP-1 reporter cell line resulted in a much stronger innate immune response as indicated by the production of IFN-sensitive response element-driven (ISRE) reporter luciferase activity (FIG. 3).

Given the ability of the VRP prototype to confer protection in a well-accepted CCHF virus mouse model, VRPs with additional genetic modification(s) of the vOTU domain are also of use. Such variants of the L protein include any one or any combination of: C40A/S/R, I13R/E/K, A129R, P77D/T, T120L, E128V, A129G, and V18I, which lead to substantial attenuation of vOTU deubiquitinase activity and/or ISG15 activity. The impact of exemplary mutants on CCHF virus vOTU is shown in FIG. 4. Portions of the nairovirus GPC can also be used. These can be generated not only for the CCHF virus African strain IbAr10200, but any one of a number of different strains, such as, but not limited to, the CCHF virus Asia (Oman1998) and Europe (Turkey2004) strains.

### Example 2

### Heterologous protection against Crimean-Congo hemorrhagic fever in mice after a single dose of replicon particle vaccine

A single-dose virus replicon particle (VRP) vaccine regimen as assessed in a mouse model for protection against Turkey or Oman strains of CCHFV. All mice were completely protected from disease, supporting broad applicability of this platform for prevention of infection with genetically diverse strains of CCHFV.

A concern for vaccine development is the high genetic diversity amongst CCHFV strains, especially between strains from different geographic regions (Carroll et al., Mol. Phylogenet. Evol. 55, 1103-10, 2010). However, all CCHFV vaccine studies to date have only investigated homologous challenge. Thus, protective efficacy against genetically diverse CCHFV strains was investigated.

First, to investigate disease progression after infection with genetically diverse strains representing two additional CCHFV clades, female B6.129S2*-Ifnar1^{tm1Agt}*/Mmjax mice (MMRRC 032045-JAX; 7-8 weeks of age) were inoculated subcutaneously (SC) in the inter-scapular region with a target dose of 1 × 10² 50% tissue culture infective dose (TCID50) of the Nigerian tick isolate (recombinant CCHFV-IbAr10200, Africa-3 clade), or with one of four low-passage clinical isolate strains representing either the Europe-1 clade (CCHFV-Turkey) or the Asia-1 clade (CCHFV-Oman-97, -Oman-98, or -UAE) (n = 5 each, Fig 7A). Mice were housed in a climate-controlled laboratory with a 12 h day/night cycle; provided sterilized commercially available mouse chow and water *ad libitum*; and group-housed on autoclaved corn cob bedding (Bed-o'Cobs^{®} ¼", Anderson Lab Bedding) with cotton nestlets in an isolator-caging system (Thoren Caging, Inc., Hazleton, PA, USA) with a HEPA-filtered inlet and exhaust air supply. Mice were humanely euthanized with isoflurane vapor at the indicated time points, or when clinical illness scores based on piloerection, behavior (i.e. reluctance to leave nest), activity level, neurological signs (i.e. ataxia, tremors, paresis/paralysis), dehydration, dyspnea, and/or weight loss (>20% from baseline at -1 dpi) indicated that the animal was in distress or in the terminal stages of disease.

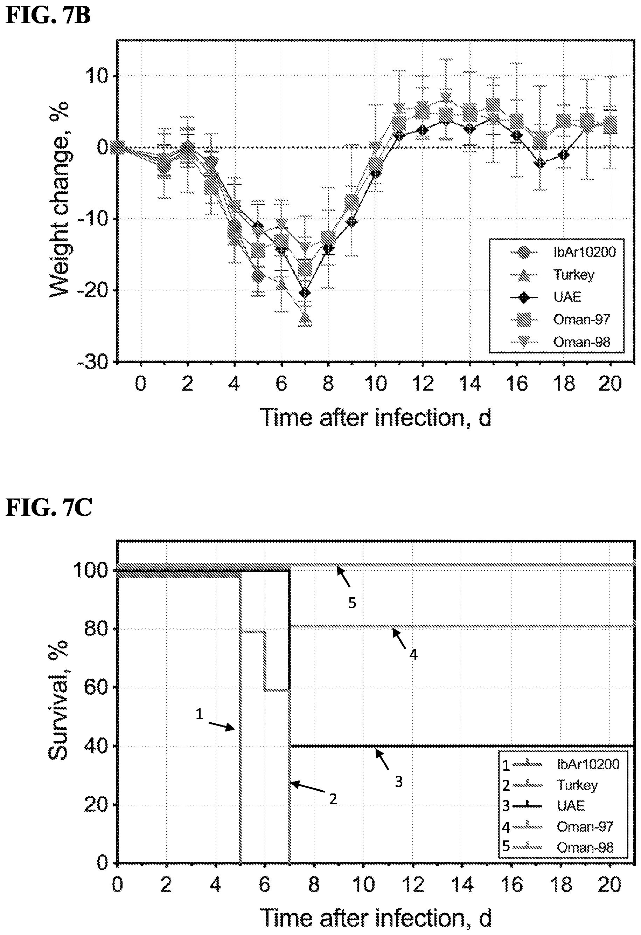

Following inoculation, all mice exhibited clinical signs beginning ~3 days post infection (dpi; Fig. 7B, 7C; Table 1).

**Table 1. Serological analyses of CCHFV-infected IFNAR^{-/-} mice.**

| **CCHFV strain** | | **DPI** | **Outcome** | **Anti-NP IgM** | **Anti-NP IgG** | **Anti-Gc IgG** |
|---|---|---|---|---|---|---|
| **IbAr10200** | 1 | 5 | Fatal | 0 | 23 | 154 |
| | 2 | 5 | Fatal | 0 | 1 | 29 |
| | 3 | 5 | Fatal | 5 | 15 | 123 |
| | 4 | 5 | Fatal | 0 | 7 | 55 |
| | 5† | 5 | Fatal | NS | NS | NS |
| **Turkey** | 1 | 5 | Fatal | 18 | 4 | 50 |
| | 2 | 7 | Fatal | 1690 | 2932 | 2985 |
| | 3‡ | 7 | Fatal | NS | 6115 | NS |
| | 4 | 7 | Fatal | 1976 | 7555 | 6011 |
| | 5 | 6 | Fatal | 165 | 148 | 214 |
| **UAE** | 1 | 21 | Survivor | 183 | 38093 | 1740 |
| | 2 | 21 | Survivor | 1441 | 39895 | 2302 |
| | 3 | 7 | Fatal | 6026 | 6090 | 15722 |
| | 4† | 7 | Fatal | NS | NS | NS |
| | 5 | 7 | Fatal | 4924 | 1840 | 2508 |
| **Oman-97** | 1 | 21 | Survivor | 352 | 19111 | 2207 |
| | 2 | 21 | Survivor | 105 | 19578 | 2469 |
| | 3 | 21 | Survivor | 85 | 38597 | 1950 |
| | 4 | 21 | Survivor | 518 | 64279 | 3065 |
| | 5 | 7 | Fatal | 5428 | 11229 | 16193 |
| **Oman-98** | 1 | 21 | Survivor | 215 | 41492 | 3880 |
| | 2 | 21 | Survivor | 413 | 100248 | 4487 |
| | 3 | 21 | Survivor | 46 | 36379 | 1266 |
| | 4 | 21 | Survivor | 40 | 36408 | 1773 |
| | 5 | 21 | Survivor | 267 | 21201 | 2253 |

| | | | | | | |
|---|---|---|---|---|---|---|
| IgM and IgG antibodies against CCHFV NP and IgG against CCHFV Gc in plasma were obtained at disease end-point or at completion of study at 21 dpi. Values presented in antibody activity units (AAU). All animals were challenged subcutaneously with 1 × 10² TCID₅₀ of the indicated CCHFV strains. †No plasma sample obtained. ‡Not enough sample to run all assays. NS, no sample. | | | | | | |

In addition to pronounced and progressive weight loss, decreased activity as observed and, in mice reaching end-stage disease, severe hypoactivity and moribundity. Clinical signs were most severe in mice infected with IbAr10200. Despite significant weight loss (up to 20% from baseline at -1 dpi), disease onset was less acute and clinical signs less pronounced in mice infected with CCHFV-Turkey, -Oman-97, -Oman-98, or -UAE than in those infected with IbAr10200, even in animals reaching end-point criteria.

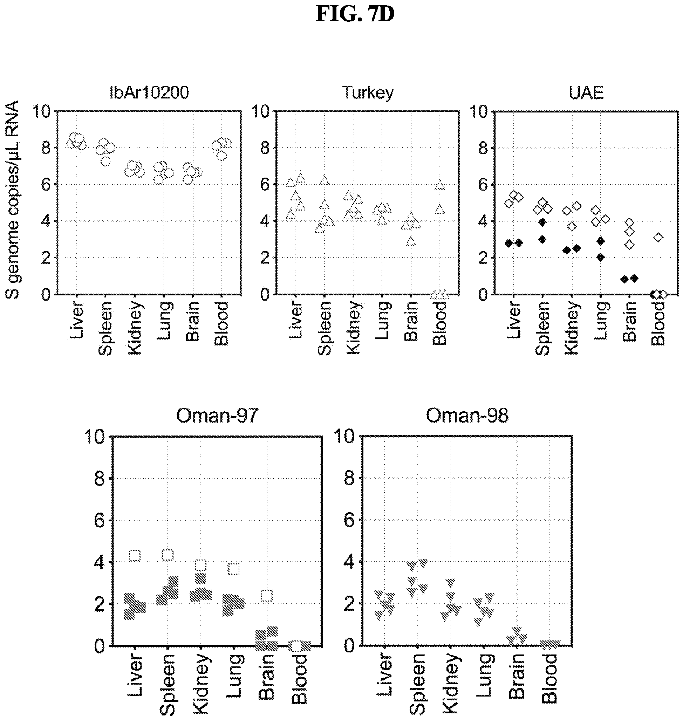

RNA was extracted from blood and homogenized tissue samples using the MAGMAX^{™}-96 Total RNA Isolation Kit (Thermo-Fisher Scientific) on a 96-well ABI MAGMAX^{™} extraction platform with a DNaseI treatment step according to manufacturer's instructions. RNA was quantitated using a one-step real-time RT-PCR targeting a strain-specific NP gene sequence (Table 2), and was standardized to 18S with a SUPERSCRIPT^{®} III Platinum One-Step qRT-PCR Kit (Thermo-Fisher Scientific) according to manufacturer's instructions. Relative viral S genome copy numbers were calculated using standards prepared from in vitro-transcribed S genomic RNA and expressed per µL of eluted RNA. As in previous reports, viral RNA was widely distributed in all mice, with levels highest in animals that succumbed early and lowest in convalescent animals (Fig 7D).

**Table 2. Primer and hydrolysis probe sequences for CCHFV strain-specific S segment (NP) RT-PCR.**

| **Strain** | | **Sequence (5'- 3')** |
|---|---|---|
| IbAr10200 | Fwd | ATGAACAGGTGGTTTGAAGAGTT (SEQ ID NO: 11) |
| | Rev | TGGCACTGGCCATCTGA (SEQ ID NO: 12) |
| | Probe | 6FAM/TGTCCAAAT/ZEN/TGGGAACACTCTCGCA/IABKFQ (SEQ ID NO: 13) |
| Oman* | Fwd | TGATGATGCTGCCTTAGGATC (SEQ ID NO: 14) |
| | Rev | TGGAGACTGTTACCAACAAGA (SEQ ID NO: 15) |
| | Probe | 6FAM/TGCAGCAGG/ZEN/TGCTCAGAGGCTACA/IABKFQ (SEQ ID NO: 16) |
| Turkey | Fwd | GGCTGAGTGTGGAGCACC (SEQ ID NO: 17) |
| | Rev | AACAGGATTTAACATACAGGACATG (SEQ ID NO: 18) |
| | Probe | 6FAM/TCCCTTGTT/ZEN/GGCAAGCAGTCTCCA/IABKFQ (SEQ ID NO: 19) |
| UAE | Fwd | ATGGAGACTGTTACCAACAAG ((SEQ ID NO: 20) |
| | Rev | TGATGATGCTGCCTTAGGATC (SEQ ID NO: 21) |
| | Probe | 6FAM/TGCAGCAGG/ZEN/TGCTCAGAGGCTACA/IABKFQ (SEQ ID NO: 22) |

| | | |
|---|---|---|
| *Primer-probe set detects both Oman-97 and Oman-98. Fwd, forward primer; Rev, reverse primer. | | |

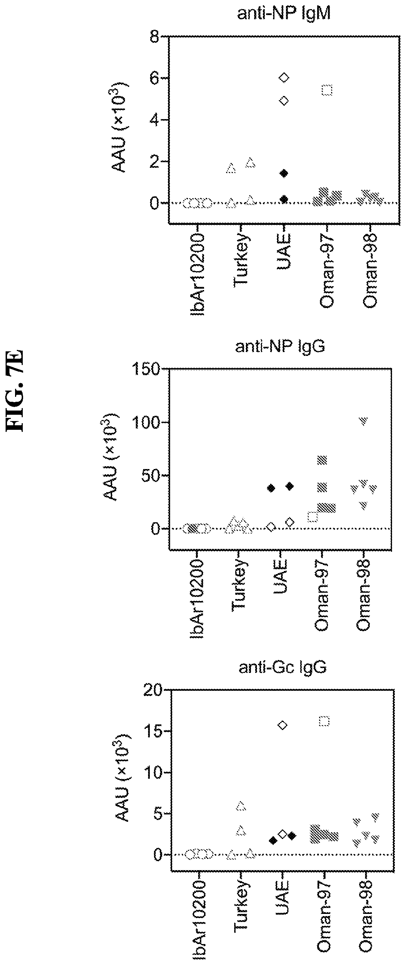

To better understand the kinetics and magnitude of antibody responses to CCHFV infection in mice, serological analyses were conducted on plasma obtained at the time of euthanasia (Fig 7E; Table 1). Plasma was separated from whole blood collected in lithium heparin tubes by centrifuging 3 min at 8000 rpm. Samples were inactivated using gamma irradiation (5 million rads from a ⁶⁰Co source). CCHFV NP IgG and IgM were detected using commercial ELISA kits (Alpha Diagnostics International, AE-320400-1 and AE-320410-1 (AP92 strain sequence)). CCHFV Gc IgG levels were determined using an in-house ELISA assay using purified CCHFV-Oman Gc ectodomain bound to nickel-coated 96-well plates (1 µg per well). OD₄₅₀ values were obtained for a 3-fold dilution series of plasma (1:100, 1:300, 1:900, 1:2700, 1:8100, 1:24300). Antibody activity units (AAU) for all assays were determined according to Alpha Diagnostics International's recommended protocol. In brief, power trendlines (y = cx^{b}) were fitted to the OD₄₅₀ values, and AAUs were calculated for each assay based on an OD₄₅₀ value determined from negative control animals (no VRP pre-bleed plasma, n = 8): anti-NP IgM = 0.35 OD₄₅₀; anti-NP IgG = 0.15 OD₄₅₀; anti-Gc IgG = 0.60 OD₄₅₀.

As expected, antibody responses varied based on time after infection and disease outcome. In fatal human CCHF cases, detectable IgM or IgG antibodies are typically not produced (Bente et al., Antiviral Res. 100, 159-89, 2013), while robust development of IgM and IgG is considered a positive prognostic indicator (Shepherd et al., Rev. Infect. Dis. 11, S801-6, 1989). Similarly, no or low antibody reactivity was detected in animals that first succumbed to disease (IbAr10200-infected mice at 5 dpi). However, in mice that succumbed at 7 dpi, more robust reactivity was detected for all antibodies assessed. In almost all survivors, IgG against both NP and glycoprotein (Gc) were detected, but not IgM, when sampled at study completion (21 dpi).

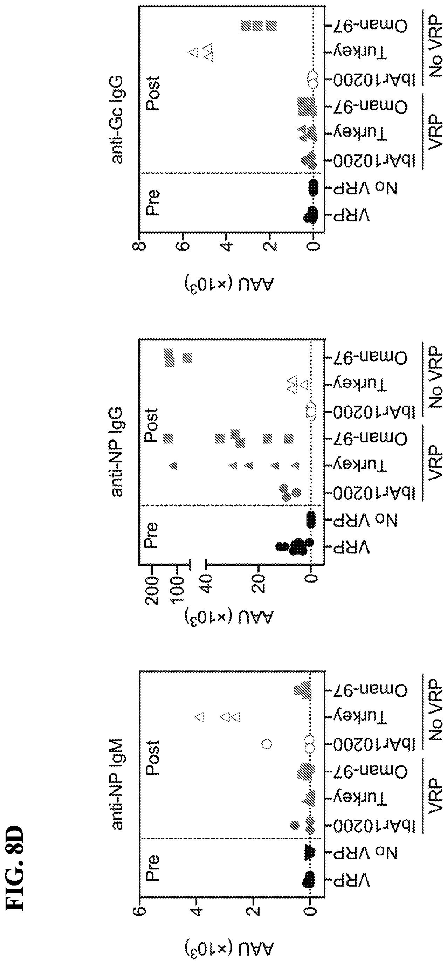

Based on these data supporting use of IFNAR^{-/-} mice infected with various CCHFV strains as alternative disease models, CCHFV-Turkey and -Oman-97 were subsequently used for challenge studies in IFNAR^{-/-} mice vaccinated with a single dose of the VRP vaccine. Female B6.12952-*Ifnar1^{tm1Agt}*/Mmjax mice (MMRRC 032045-JAX; 6 weeks of age), housed as above, were vaccinated SC in the inter-scapular region with DMEM (mock, n = 3, each strain) or a target dose of 1 × 10⁵ TCID₅₀ of CCHFV-VRP (n = 3 for IbAr10200, or n = 6 for Turkey or Oman); back-titer dose: 2.15 × 10⁵ TCID₅₀) (Fig 8A). Consistent with our previous report (Scholte et al., 2019) and the safety profile we observed in suckling mice inoculated intracranially with VRP (Fig. 9), no clinical signs were observed in VRP-vaccinated IFNAR^{-/-} mice during the post-vaccination period. Samples were collected 24 days post vaccination to assess antibody levels in plasma prior to challenge (Fig. 8D; Table 3). All vaccinated animals had detectable levels of anti-NP IgG, a subset had evidence of low-level anti-Gc IgG activity, and only a few had minimal anti-NP IgM antibody activity at the time of sampling.

**Table 3. Serology and associated outcome post challenge in CCHFV VRP-vaccinated IFNAR^{-/-} mice.**

| **Vaccine status + challenge strain** | | **DPI** | **Outcome** | **Anti-NP IgM** | | **Anti-NP IgG** | | **Anti-Gc IgG** | |
|---|---|---|---|---|---|---|---|---|---|
| | | | | **Pre** | **Post** | **Pre** | **Post** | **Pre** | **Post** |
| **VRP + IbAr10200** | 1 | 21 | Survivor | 0 | 0 | 3174 | 10441 | 23 | 90 |
| | 2 | 21 | Survivor | 4 | 6 | 4291 | 9375 | 14 | 63 |
| | 3 | 21 | Survivor | 122 | 537 | 5363 | 5523 | 114 | 362 |
| **VRP + Oman-97** | 1 | 21 | Survivor | 0 | 162 | 6045 | 135840 | 16 | 525 |
| | 2 | 21 | Survivor | 109 | 287 | 11946 | 26918 | 44 | 129 |
| | 3 | 21 | Survivor | 55 | 89 | 4154 | 16563 | 52 | 52 |
| | 4 | 21 | Survivor | 7 | 104 | 4915 | 28952 | 218 | 503 |
| | 5 | 21 | Survivor | 71 | 194 | 3998 | 8575 | 67 | 193 |
| | 6 | 21 | Survivor | 11 | 8 | 9956 | 34684 | 31 | 136 |
| **VRP + Turkey** | 1 | 21 | Survivor | 0 | 2 | 4633 | 24000 | 17 | 39 |
| | 2 | 21 | Survivor | 4 | 8 | 3526 | 6313 | 73 | 111 |
| | 3 | 21 | Survivor | 7 | 4 | 6591 | 29946 | 24 | 126 |
| | 4 | 21 | Survivor | 0 | 19 | 5384 | 364211 | 54 | 566 |
| | 5 | 21 | Survivor | 13 | 1 | 4762 | 14085 | 271 | 210 |
| | 6 | 21 | Survivor | 34 | 171 | 660 | 120267 | 47 | 541 |
| **No VRP + IbAr10200** | 1 | 4 | Fatal | NS | 1530 | NS | 0 | NS | 76 |
| | 2 | 4 | Fatal | 0 | 26 | 0 | 0 | 1 | 41 |
| | 3 | 4 | Fatal | 0 | 16 | 3 | 2 | 0 | 19 |
| **No VRP + Oman-97** | 1 | 21 | Survivor | 0 | 117 | 11 | 129585 | 15 | 1945 |
| | 2 | 21 | Survivor | 0 | 402 | 4 | 133942 | 15 | 2579 |
| | 3 | 21 | Survivor | 2 | 161 | 4 | 59920 | 6 | 3111 |
| **No VRP + Turkey** | 1 | 7 | Fatal | 4 | 3922 | 3 | 3279 | 10 | 4891 |
| | 2 | 7 | Fatal | 0 | 2654 | 1 | 7363 | 9 | 5551 |
| | 3 | 7 | Fatal | 0 | 3019 | 0 | 7367 | 8 | 4833 |

| | | | | | | | | | |
|---|---|---|---|---|---|---|---|---|---|
| IgM and IgG antibodies against CCHFV NP and IgG against CCHFV Gc in plasma obtained 24 days after vaccination (Pre), or at disease end-point or completion of study at 21 dpi (Post). Values presented in AAU. All animals were challenged subcutaneously with 1 × 10² TCID₅₀ of indicated CCHFV strains 28 days post VRP vaccination (1 × 10⁵ TCID₅₀) or mock vaccination (No VRP). Pre-challenge blood samples from unvaccinated mice (No VRP) were used to determine cut-off values. N/A, not applicable; NS, no sample. | | | | | | | | | |

At 28 days post vaccination, groups of mice (n = 6 for recombinant CCHFV-IbAr10200, or n = 9 for CCHFV-Turkey or CCHFV-Oman-97) were challenged with a target dose of 1 × 10² TCID₅₀ of indicated CCHFV strain (back-titer dose: 3.73 × 10² TCID₅₀). Mice were humanely euthanized as above, with weight loss end-point criteria extended to >25% (from baseline at -1 dpi). Post-challenge, all unvaccinated mice developed clinical signs (weight loss, decreased water consumption, hunched posture, hypoactivity) and reached end-point criteria (CCHFV-IbAr 10200 or -Turkey) or recovered from disease by ~12 dpi (CCHFV-Oman-97) (Fig 2B-C, Fig. 10). In contrast, all vaccinated mice were protected from both disease and death, demonstrating heterologous protection from CCHF disease in single-dose VRP-vaccinated IFNAR^{-/-} mice. An increase in plasma anti-NP and anti-Gc IgG activity was observed in almost all VRP-vaccinated mice post challenge. In unvaccinated mice, both antibody and viral RNA levels were comparable to those detected in strain comparison studies (Fig. 8D, Fig. 11).

A variety of CCHFV vaccine candidates has been screened in immunodeficient mouse models. The majority of these use a prime/boost vaccination approach. The only reported vaccine with a single dose regimen was a human adenovirus 5-vectored vaccine expressing the NP protein, which conferred 33% protection (Zivcec et al., PLoS Negl. Trop. Dis. 9, 1-23, 2018). Recently, use of a single dose of VSV-based vaccine was shown to provide protection from lethal outcome, but did not protect all mice from clinical disease (Rodriguez et al., Hemorrhagic Fever. Sci. Rep. 9, 7755, 2019). While a number of vaccines based on viral NP antigen alone have demonstrated efficacy (reviewed in (Dowall et al., Hum. Vaccines Immunother. 12, 519-27, 2016)), to date, none of the CCHFV vaccine platforms expressing NP alone (modified Vaccinia virus Ankara (MVA) (Dowall et al., Hum. Vaccines Immunother. 12, 519-27, 2016) or human adenovirus 5 (Zivcec et al., PLoS Negl. Trop. Dis. 12, e0006628, 2018)) conferred complete protection against lethal disease. In addition to the presently disclosed VRP platform, four vaccines strategies have been reported to confer complete protection against lethal CCHFV in IFNAR^{-/-} mice: (1) an MVA-based vaccine vector expressing the full-length glycoproteins (MVA-GPC) (Buttigieg et al., PLoS One 9, e91516, 2014); (2) plasmid DNA vaccination (NP, Gn, and Gc) (Hinkula et al., J. Virol. 91, JVI.02076-16, 2017); (3) adenovirus 5 expressing NP (Aligholipour Farzani et al., Viruses 11, 237, 2019); and; (4) a bovine herpes type vector expressing NP (Aligholipour Farzani et al., Viruses 11, 237, 2019). Notably, all these vaccines were administered with one or more booster doses prior to challenge.

Both antibody and T-cell responses have been indicated as required for protection; in follow-up studies using the MVA-GPC vaccine, transfer of both T-cells and antibodies was apparently required for protection in mice (Dowall et al., PLoS One 11, 1-13, 2016). In contrast, vaccine studies indicate that the production of neutralizing antibodies does not correspond to efficacy (Hinkula et al., J. Virol. 91, JVI.02076-16, 2017; Kortekaas et al., Vector-Borne Zoonotic Dis. 15, 759-64, 2015). In this study, protection to a Gc-specific antibody response cannot be correlated, as Gc-antibodies were not detected in all vaccinated mice. This may represent an absence of response, or may be due to limitations of the assay. However, NP-specific antibodies were detected in all vaccinated mice, an antigen actively produced by the VRP, as opposed to Gc, which is only present on the VRP surface. While this correlates with results from other vaccine studies demonstrating that NP antigen alone can elicit protective responses, protective antibodies may also target other non-Gc GPC-derived antigens not captured in the disclosed analyses (e.g., Gn, GP38). The VRP platform was demonstrated to also protect against additional diverse CCHFV strains.

### Example 3

### Additional Methods

*Viruses:* For CCHFV strain comparison or heterologous challenge experiments, mice were inoculated with Turkey-200406546 (GENBANK^{®} Accession Nos. KY362517, KY362519, KY362515), UAE-199812347 (GenBank: MF289419, MF289418, MF289417), Oman-199723179, Oman-199809166 (GENBANK^{®} Accession Nos. KY362516, KY362518, KY362514), or recombinant IbAr10200 based on wild-type IbAr10200 (GENBANK^{®} Accession Nos. KJ648914, KJ648915, and KJ648913) (Bergeron et al., PLoS Pathog. 11, e1004879, 2015). Passage histories for stocks used in strain comparison experiments: recombinant IbAr10200: rescued in Huh7 cells, passaged 3× in BSR-T7/5 cells; Turkey: 1× suckling mouse brain, 1× SW-13 cells; UAE: 2× Vero-E6, 1× SW-13 cells; Oman-97: 1× Vero-E6, 1× SW-13 cells; and Oman-98: 2× Vero-E6, 1× SW-13 cells. Passage histories for stocks used in heterologous protection experiments: recombinant IbAr10200: as above; Oman-97: 1× suckling mouse brain, 1× BSR-T7/5 cells; and Turkey: 1× suckling mouse brain, 1× BSR-T7/5 cells. Viral titers were calculated as 50 percent tissue culture infective dose (TCID50) (Reed and Muench, 1938), and were determined in parallel in either SW-13 cells by observing cytopathic effects, or in BSR-T7/5 cells by measuring indirect immunofluorescence (Scholte et al., Cell Rep. 20, 2396-2407, 2017).. All virus stocks were verified by next-generation sequencing and confirmed mycoplasma free.

*VRP production and titration:* Six-well plates were seeded with 3.5 × 10⁵ Huh7 cells/well 1 day prior to transfection in 3 mL of DMEM supplemented with 1% non-essential amino acids, 1 mM sodium pyruvate, and 10% FBS. 16-24 h later, cells were transfected with pT7-S (1 µg), pT7-L (1 µg), pCAGGS-L (0.33 µg), pCAGGS-NP (0.66 µg), pCAGGS-GPC-Oman (1 µg), and pCAGGS-T7 (1 µg), combined with 12.5 µL of Mirus LT1 transfection reagent (Mirus Bio, Madison, WI, USA) in 250 µL of OPTI-MEM (Life Technologies, Grand Island, NY, USA). Supernatants containing VRPs were harvested 4-5 days post transfection. VRP stocks were titrated by TCID₅₀ on BSR/T7 cells (Reed and Muench, Am. J. Hyg. 27, 493-497, 1938). Positive wells were scored based on the detection of at least one CCHFV NP positive cells detectable by immunofluorescence using a rabbit anti-NP antibody (#04-0011, IBT Bioservices) and Alexa-488 goat anti-rabbit secondary antibody.

*Phylogenetics:* The full-length S genome segment sequences of CCHFV strains from each of the seven clades (Africa 1, Africa 2, Africa 3, Asia 1, Asia 2, Europe 1, and Europe 2) were aligned and a phylogenetic tree was constructed (neighbor joining tree construction, Jukes-Cantor distance measure, and ×1000 bootstrap measurements) using CLC Genomics Workbench version 9.5.2. Radial phylogenetic tree was drawn using FigTree version 1.4.3.

## Claims

1. A Crimean-Congo hemorrhagic fever (CCHF) virus replicon particle (VRP), comprising:
(i) CCHF virus Gn and Gc glycoproteins;
(ii) CCHF virus L protein;
(iii) CCHF virus nucleoprotein;
(iv) a CCHF virus L genome segment; and
(v) a CCHF virus S genome segment,
wherein the CCHF VRP a) does not contain a CCHF virus M genome segment or b) encodes a domain of a glycoprotein precursor (GPC) but not a full-length GPC.

2. The CCHF VRP of claim 1, wherein the CCHF VRP comprises an M genome segment that encodes the domain of the GPC but not the full-length GPC, and wherein the domain is a mucin-like domain, GP38 domain, mucin-like+GP38 domain, or an NsM, Gn, Gc receptor binding domain.

3. The CCHF VRP of claim 1 or claim 2, wherein the CCHF virus is African strain IbAr10200, or wherein the CCHF virus is CCHF virus Asia (Oman1998) strain or Europe (Turkey2004) strain.

4. The CCHF VRP of any one of claims 1-3, wherein the L genome segment encodes a viral ovarian tumor domain protease (vOTU) comprising one or more mutations, wherein the one or more mutations disrupt vOTU deubiquitinase activity and/or interferon-simulated gene product 15 (ISG15) activity, particularly wherein the one or more mutations comprise a Q16R mutation, numbered with reference to SEQ ID NO: 8, and wherein vOTU deubiquitinase activity is disrupted.

5. The CCHF VRP of claim 4, wherein the one or more mutations comprise or further comprise at least one of I13R/E/K, V18I, C40A/S/R, P77D/T, T120L, E128V and A129R/G, numbered with reference to SEQ ID NO: 8.

6. The CCHF VRP of any one of claims 1-5, wherein the amino acid sequence of the L protein is at least 90% identical to SEQ ID NO: 7 or SEQ ID NO: 8, particularly wherein the amino acid sequence of the L protein is at least 95% identical to SEQ ID NO: 7 or SEQ ID NO: 8, particularly wherein the amino acid sequence of the L protein comprises SEQ ID NO: 7 or SEQ ID NO: 8;
and/or wherein the amino acid sequence of GPC is at least 90% identical to SEQ ID NO: 9, particularly wherein the amino acid sequence of GPC is at least 95% identical to SEQ ID NO: 9, particularly wherein the amino acid sequence of GPC comprises SEQ ID NO: 9;
and/or wherein the amino acid sequence of the nucleoprotein is at least 90% identical to SEQ ID NO: 6, particularly wherein the amino acid sequence of the nucleoprotein is at least 95% identical to SEQ ID NO: 6, particularly wherein the amino acid sequence of the nucleoprotein comprises SEQ ID NO: 6;
and/or wherein the L genome segment comprises a nucleotide sequence at least 90% identical to nucleotides 2706-14865 of SEQ ID NO: 2, particularly wherein the L genome segment comprises a nucleotide sequence at least 95% identical to nucleotides 2706-14865 of SEQ ID NO: 2, particularly wherein the L genome segment comprises the nucleotide sequence of nucleotides 2706-14865 of SEQ ID NO: 2, particularly wherein the S genome segment comprises a nucleotide sequence at least 90% identical to nucleotides 2706-4377 of SEQ ID NO: 1;
and/or wherein the S genome segment comprises a nucleotide sequence at least 95% identical to nucleotides 2706-4377 of SEQ ID NO: 1, particularly wherein the S genome segment comprises the nucleotide sequence of nucleotides 2706-4377 of SEQ ID NO: 1.

7. The CCHF VRP of any one of claims 1-6, wherein the S genome segment comprises a CCHF virus nucleoprotein open reading frame (ORF) and a heterologous ORF, particularly wherein the heterologous ORF encodes a portion of a CCHF virus GPC, or wherein the heterologous ORF encodes a fluorescent protein, and/or wherein the nucleoprotein ORF and the heterologous ORF are in-frame and are separated by the coding sequence for a self-cleaving 2A peptide, particularly wherein the 2A peptide comprises a porcine teschovirus-1 (PTV1) 2A (P2A) peptide, a foot and mouth disease virus (FMDV) 2A (F2A) peptide, an equine rhinitis A virus (ERAV) 2A (E2A) peptide or a Thosea asigna virus (TaV) 2A (T2A) peptide.

8. A method of producing CCHF VRP, comprising:
transfecting a host cell with:
a plasmid comprising an antigenomic copy of a CCHF virus L segment;
a plasmid comprising an antigenomic copy of a CCHF virus S segment;
a plasmid encoding CCHF virus glycoprotein (GPC);
a plasmid encoding a CCHF virus nucleoprotein;
a plasmid encoding a CCHF virus L protein; and
culturing the cells for a period of time sufficient to produce CCHF VRP comprising a CCHF virus L genome segment, a CCHF virus S genome segment, the GPC, or a portion thereof, the nucleoprotein and the L protein.

9. The method of claim 8, further comprising collecting the CCHF VRP from the cell culture supernatant, particularly further comprising amplifying the CCHF VRP in a susceptible cell line expressing CCHF virus GPC in trans.

10. The method of claim 8 or 9, wherein the CCHF virus is African strain IbAr10200, or wherein the CCHF virus is CCHF virus Asia (Oman1998) strain or Europe (Turkey2004) strain.

11. The method of any one of claims 8-10, wherein the L genome segment encodes a viral ovarian tumor domain protease (vOTU) comprising one or more mutations, wherein the one or more mutations disrupt vOTU deubiquitinase activity and/or interferon-stimulated gene (ISG15) activity, particularly wherein the one or more mutations comprise a Q16R mutation, numbered with reference to SEQ ID NO: 8, and wherein vOTU deubiquitinase activity is disrupted, and/or wherein the one or more mutations comprise or further comprise at least one of I13R/E/K, V18I, C40A/S/R, P77D/T, T120L, E128V and A129R/G, numbered with reference to SEQ ID NO: 8.

12. The method of any one of claims 8-11, wherein the plasmid comprising an antigenomic copy of the S segment and the plasmid comprising an antigenomic copy of the L segment further comprise a T7 promoter operably linked at the 5' end of the antigenomic copy and a hepatitis delta virus ribozyme at the 3' end of the antigenomic copy.

13. The method of any one of claims 8-12, wherein:
(i) the plasmid comprising an antigenomic copy of the L segment comprises a nucleotide sequence that is at least 90% or at least 95% identical to SEQ ID NO: 2;
(ii) the plasmid comprising an antigenomic copy of the S segment comprises a nucleotide sequence that is at least 90% or at least 95% identical to SEQ ID NO: 1;
(iii) the plasmid encoding the CCHF virus GPC is at least 90% or at least 95% identical to SEQ ID NO: 5;
(v) the plasmid encoding the CCHF virus nucleoprotein comprises a nucleotide sequence that is at least 90% or at least 95% identical to SEQ ID NO: 3; and/or
(vi) the plasmid encoding the CCHF virus L protein comprises a nucleotide sequence that is at least 90% or at least 95% identical to SEQ ID NO: 4, particularlywherein:
(i) the nucleotide sequence of the plasmid comprising an antigenomic copy of the L segment comprises SEQ ID NO: 2;
(ii) the nucleotide sequence of the plasmid comprising an antigenomic copy of the S segment comprises SEQ ID NO: 1;
(iii) the nucleotide sequence of the plasmid encoding the CCHF virus GPC comprises SEQ ID NO: 5;
(iv) the nucleotide sequence of the plasmid encoding the CCHF virus nucleoprotein comprises SEQ ID NO: 3; and/or
(v) the nucleotide sequence of the plasmid encoding the CCHF virus L protein comprises SEQ ID NO: 4.

14. An immunogenic composition comprising the CCHF VRP of any one of claims 1-7, and a pharmaceutically acceptable carrier, particularly further comprising an adjuvant.

15. The immunogenic composition of claim 14 for use in a method of eliciting an immune response against CCHF virus or a CCHF viral protein in a subject or of immunizing a subject against CCHF virus infection, comprising administering to the subject an effective amount of the immunogenic composition,
particularly wherein the viral protein is a CCHF virus nucleoprotein or a CCHF virus L protein,
and/or wherein the subject is human,
and/or wherein only one dose of the immunogenic composition is administered to the subj ect,
and/or wherein the immunogenic composition is administered intravenously, intramuscularly or subcutaneously,
and/or wherein the method induces an immune response to more than one strain of CCHF virus.

## Patentansprüche

1. Virusreplikonpartikel (VRP) des hämorrhagischen Krim-Kongo-Fiebers (CCHF), umfassend:
(i) CCHF-Virus-Gn- und Gc-Glykoproteine;
(ii) CCHF-Virus-L-Protein;
(iii) CCHF-Virus-Nukleoprotein;
(iv) ein CCHF-Virus-L-Genomsegment und
(v) ein CCHF-Virus-S-Genomsegment, wobei das CCHF-VRP a) kein CCHF-Virus-M-Genomsegment enthält oder b) eine Domäne eines Glykoprotein-Vorläufers (GPC), jedoch nicht einen GPC voller Länge codiert.

2. CCHF-VRP nach Anspruch 1, wobei das CCHF-VRP ein M-Genomsegment umfasst, das die Domäne des GPC, jedoch nicht den GPC voller Länge codiert, und wobei die Domäne eine Mucin-ähnliche Domäne, GP38-Domäne, Mucin-ähnliche + GP38-Domäne oder eine NsM-, Gn-, Gc-Rezeptor-bindende Domäne ist.

3. CCHF-VRP nach Anspruch 1 oder Anspruch 2, wobei das CCHF-Virus der afrikanische Stamm IbAr10200 ist oder wobei das CCHF-Virus der asiatische (Oman1988) oder der europäische (Turkey2004) CCHF-Virus-Stamm ist.

4. CCHF-VRP nach einem der Ansprüche 1-3, wobei das L-Genomsegment eine virale Eierstocktumor-Domänenprotease (vOTU) codiert, die eine oder mehrere Mutationen umfasst, wobei die eine oder die mehreren Mutationen die vOTU-Deubiquitinase-Aktivität und/oder die Interferon-stimulierte Genprodukt-15-Aktivität (ISG15-Aktivität) stört, insbesondere wobei die eine oder die mehreren Mutationen eine Q16R-Mutation umfassen, unter Bezugnahme auf SEQ ID Nr. 8 nummeriert, und wobei die vOTU-Deubiquitinase-Aktivität gestört wird.

5. CCHF-VRP nach Anspruch 4, wobei die eine oder die mehreren Mutationen mindestens eine von I13R/E/K, V18I, C40A/S/R, P77D/T, T120L, E128V und A129R/G umfassen oder weiterhin umfassen, unter Bezugnahme auf SEQ ID Nr. 8 nummeriert.

6. CCHF-VRP nach einem der Ansprüche 1-5, wobei die Aminosäuresequenz des L-Proteins zu mindestens 90 % mit SEQ ID Nr. 7 oder SEQ ID Nr. 8 identisch ist, insbesondere wobei die Aminosäuresequenz des L-Proteins zu mindestens 95 % mit SEQ ID Nr. 7 oder SEQ ID Nr. 8 identisch ist, insbesondere wobei die Aminosäuresequenz des L-Proteins SEQ ID Nr. 7 oder SEQ ID Nr. 8 umfasst;
und/oder wobei die Aminosäuresequenz des GPC zu mindestens 90 % mit SEQ ID Nr. 9 identisch ist, insbesondere wobei die Aminosäuresequenz des GPC zu mindestens 95 % mit SEQ ID Nr. 9 identisch ist, insbesondere wobei die Aminosäuresequenz des GPC SEQ ID Nr. 9 umfasst;
und/oder wobei die Aminosäuresequenz des Nukleoproteins zu mindestens 90 % mit SEQ ID Nr. 6 identisch ist, insbesondere wobei die Aminosäuresequenz des Nukleoproteins zu mindestens 95 % mit SEQ ID Nr. 6 identisch ist, insbesondere wobei die Aminosäuresequenz des Nukleoproteins SEQ ID Nr. 6 umfasst;
und/oder wobei das L-Genomsegment eine Nukleotidsequenz umfasst, die zu mindestens 90 % mit den Nukleotiden 2706-14.865 von SEQ ID Nr. 2 identisch ist, insbesondere wobei das L-Genomsegment eine Nukleotidsequenz umfasst, die zu mindestens 95 % mit den Nukleotiden 2706-14.865 von SEQ ID Nr. 2 identisch ist, insbesondere wobei das L-Genomsegment die Nukleotidsequenz der Nukleotide 2706-14.865 von SEQ ID Nr. umfasst, insbesondere wobei das S-Genomsegment eine Nukleotidsequenz umfasst, die zu mindestens 90 % mit den Nukleotiden 2706-4377 von SEQ ID Nr. 1 identisch ist;
und/oder wobei das S-Genomsegment eine Nukleotidsequenz umfasst, die zu mindestens 95 % mit den Nukleotiden 2706-4377 von SEQ ID Nr. 1 identisch ist, insbesondere wobei das S-Genomsegment die Nukleotidsequenz der Nukleotide 2706-4377 von SEQ ID Nr. 1 umfasst.

7. CCHF-VRP nach einem der Ansprüche 1-6, wobei das S-Genomsegment ein offenes Leseraster (ORF) eines CCHF-Virus-Nukleoproteins und ein heterologes ORF umfasst, insbesondere wobei das heterologe ORF einen Abschnitt eines CCHF-Virus-GPC codiert oder wobei das heterologe ORF ein fluoreszierendes Protein codiert und/oder wobei das Nukleoprotein-ORF und das heterologe ORF in-frame sind und durch die codierende Sequenz für ein selbstspaltendes 2A-Peptid getrennt sind, insbesondere wobei das 2A-Peptid ein Teschovirus-1-(PTV1)-2A-(P2A)-Peptid des Schweins, ein Maul- und Klauenseuche-Virus-(FMOV)-2A-(F2A)-Peptid, ein Rhinitus-A-Virus-(ERAV)-2A-(E2A)-Peptid des Pferds oder ein Thosea-asigna-Virus-(TaV)-2A-(T2A)-Peptid umfasst.

8. Verfahren zur Produktion eines CCHF-VRP, umfassend: Transfizieren einer Wirtszelle mit:
einem Plasmid, das eine antigenomische Kopie eines CCHF-Virus-L-Segments umfasst;
einem Plasmid, das eine antigenomische Kopie eines CCHF-Virus-S-Segments umfasst;
einem Plasmid, das ein CCHF-Virus-Glykoprotein (GPC) codiert;
einem Plasmid, das ein CCHF-Virus-Nukleoprotein codiert;
einem Plasmid, das ein CCHF-Virus-L-Protein codiert; und
Kultivieren der Zellen für einen Zeitraum, der ausreicht, um ein CCHF-VRP zu produzieren, das ein CCHF-Virus-L-Genomsegment, ein CCHF-Virus-S-Genomsegment, das GPC oder einen Abschnitt davon, das Nukleoprotein und das L-Protein umfasst.

9. Verfahren nach Anspruch 8, weiterhin umfassend ein Sammeln des CCHF-VRP aus dem Zellkulturüberstand, insbesondere weiterhin umfassend ein Amplifizieren des CCHF-VRP in einer empfänglichen Zelllinie, die das CCHF-Virus-GPC in trans exprimiert.

10. Verfahren nach Anspruch 8 oder 9, wobei das CCHF-Virus der afrikanische Stamm IbAr10200 ist oder wobei das CCHF-Virus der asiatische (Oman1988) oder der europäische (Turkey2004) CCHF-Virus-Stamm ist.

11. Verfahren nach einem der Ansprüche 8-10, wobei das L-Genomsegment eine virale Eierstocktumor-Domänenprotease (vOTU) codiert, die eine oder mehrere Mutationen umfasst, wobei die eine oder die mehreren Mutationen die vOTU-Deubiquitinase-Aktivität und/oder die Interferon-stimulierte Gen-Aktivität (ISG15-Aktivität) stört, insbesondere wobei die eine oder die mehreren Mutationen eine Q16R-Mutation umfassen, unter Bezugnahme auf SEQ ID Nr. 8 nummeriert, und wobei die vOTU-Deubiquitinase-Aktivität gestört wird, und/oder wobei die eine oder die mehreren Mutationen mindestens eine von I13R/E/K, V18I, C40A/S/R, P77D/T, T120L, E128V und A129R/G umfassen oder weiterhin umfassen, unter Bezugnahme auf SEQ ID Nr. 8 nummeriert.

12. Verfahren nach einem der Ansprüche 8-11, wobei das Plasmid, das eine antigenomische Kopie des S-Segments umfasst, und das Plasmid, das eine antigenomische Kopie des L-Segments umfasst, weiterhin einen T7-Promotor, der operabel mit dem 5'-Ende der antigenomischen Kopie verknüpft ist, und ein Hepatitis-Delta-Virus-Ribozym am 3'-Ende der antigenomischen Kopie umfassen.

13. Verfahren nach einem der Ansprüche 8-12, wobei:
(i) das Plasmid, das eine antigenomische Kopie des L-Segments umfasst, eine Nukleotidsequenz umfasst, die zu mindestens 90 % oder mindestens 95 % mit SEQ ID Nr. 2 identisch ist;
(ii) das Plasmid, das eine antigenomische Kopie des S-Segments umfasst, eine Nukleotidsequenz umfasst, die zu mindestens 90 % oder mindestens 95 % mit SEQ ID Nr. 1 identisch ist;
(iii) das Plasmid, das das CCHF-Virus-GPC codiert, zu mindestens 90 % oder mindestens 95 % mit SEQ ID Nr. 5 identisch ist;
(v) das Plasmid, das das CCHF-Virus-Nukleoprotein codiert, eine Nukleotidsequenz umfasst, die zu mindestens 90 % oder mindestens 95 % mit SEQ ID Nr. 3 identisch ist; und/oder
(vi) das Plasmid, das das CCHF-Virus-L-Protein codiert, eine Nukleotidsequenz umfasst, die zu mindestens 90 % oder mindestens 95 % mit SEQ ID Nr. 4 identisch ist, insbesondere wobei:
(i) die Nukleotidsequenz des Plasmids, das eine antigenomische Kopie des L-Segments umfasst, SEQ ID Nr. 2 umfasst;
(ii) die Nukleotidsequenz des Plasmids, das eine antigenomische Kopie des S-Segments umfasst, SEQ ID Nr. 1 umfasst;
(iii) die Nukleotidsequenz des Plasmids, das das CCHF-Virus-GPC codiert, SEQ ID Nr. 5 umfasst;
(iv) die Nukleotidsequenz des Plasmids, das das CCHF-Virus-Nukleoprotein codiert, SEQ ID Nr. 3 umfasst; und/oder
(v) die Nukleotidsequenz des Plasmids, das das CCHF-Virus-L-Protein codiert, SEQ ID Nr. 4 umfasst.

14. Immunogene Zusammensetzung, umfassend das CCHF-VRP nach einem der Ansprüche 1-7 und einen pharmazeutisch unbedenklichen Träger, insbesondere weiterhin umfassend ein Adjuvans.

15. Immunogene Zusammensetzung nach Anspruch 14 zur Verwendung in einem Verfahren zur Auslösung einer Immunantwort auf ein CCHF-Virus oder ein virales CCHF-Protein in einem Probanden oder zur Immunisierung eines Probanden gegen eine CCHF-VirusInfektion, umfassend ein Verabreichen einer wirksamen Menge der immunogenen Zusammensetzung an den Probanden,
insbesondere wobei das virale Protein ein CCHF-Virus-Nukleoprotein oder ein CCHF-Virus-L-Protein ist
und/oder wobei der Proband menschlich ist
und/oder wobei nur eine Dosis der immunogenen Zusammensetzung an den Probanden verabreicht wird
und/oder wobei die immunogene Zusammensetzung intravenös, intramuskulär oder subkutan verabreicht wird
und/oder wobei das Verfahren eine Immunantwort auf mehr als einen Stamm des CCHF-Virus induziert.

## Revendications

1. Particule de réplicon du virus (PRV) de la fièvre hémorragique de Crimée-Congo (FHCC), comprenant :
(i) les glycoprotéines Gn et Gc du virus FHCC ;
(ii) la protéine L du virus FHCC ;
(iii) la nucléoprotéine du virus FHCC ;
(iv) un segment L du génome du virus FHCC ; et
(v) un segment S du génome du virus FHCC, la PRV de la FHCC a) ne contenant pas de segment M du génome du virus FHCC ou b) codant un domaine d'un précurseur des glycoprotéines (GPC) mais pas un GPC de pleine longueur.

2. PRV de la FHCC selon la revendication 1, la PRV de la FHCC comprenant un segment M du génome qui code le domaine du GPC mais pas le GPC de pleine longueur, et le domaine étant un domaine mucin-like, un domaine GP38, un domaine mucin-like+GP38, ou un domaine de liaison aux récepteurs de NsM, Gn et Gc.

3. PRV de la FHCC selon la revendication 1 ou la revendication 2, le virus FHCC étant la souche africaine IbAr10200, ou le virus FHCC étant la souche asiatique (Oman1998) ou la souche européenne (Turkey2004) du virus FHCC.

4. PRV de la FHCC selon l'une quelconque des revendications 1 à 3, dans laquelle le segment L du génome code une protéase de domaine de tumeur ovarienne virale (vOTU) comprenant une ou plusieurs mutations, les une ou plusieurs mutations perturbant l'activité désubiquitinase de la vOTU et/ou l'activité du produit génique stimulé par l'interféron 15 (ISG15), en particulier dans laquelle les une ou plusieurs mutations comprennent une mutation Q16R, numérotée en référence à la SÉQ. ID n°8, et dans laquelle l'activité désubiquitinase de la vOTU est perturbée.

5. PRV de la FHCC selon la revendication 4, dans laquelle les une ou plusieurs mutations comprennent ou comprennent en outre au moins l'une de I13R/E/K, V18I, C40A/S/R, P77D/T, T120L, E128V et A129R/G, numérotées en référence à la SÉQ. ID n° 8.

6. PRV de la FHCC selon l'une quelconque des revendications 1 à 5, dans laquelle la séquence d'acides aminés de la protéine L est au moins 90 % identique à la SÉQ. ID n° 7 ou la SÉQ. ID n° 8, en particulier dans laquelle la séquence d'acides aminés de la protéine L est au moins 95 % identique à la SÉQ. ID n° 7 ou la SÉQ. ID n° 8, en particulier dans laquelle la séquence d'acides aminés de la protéine L comprend la SÉQ. ID n° 7 ou la SÉQ. ID n° 8 ;
et/ou dans laquelle la séquence d'acides aminés du GPC est au moins 90 % identique à la SÉQ. ID n° 9, en particulier dans laquelle la séquence d'acides aminés du GPC est au moins 95 % identique à la SÉQ. ID n° 9, en particulier dans laquelle la séquence d'acides aminés du GPC comprend la SÉQ. ID n° 9 ;
et/ou dans laquelle la séquence d'acides aminés de la nucléoprotéine est au moins 90 % identique à la SÉQ. ID n° 6, en particulier dans laquelle la séquence d'acides aminés de la nucléoprotéine est au moins 95 % identique à la SÉQ. ID n° 6, en particulier dans laquelle la séquence d'acides aminés de la nucléoprotéine comprend la SÉQ. ID n° 6 ;
et/ou dans laquelle le segment L du génome comprend une séquence nucléotidique au moins 90 % identique aux nucléotides 2706-14865 de la SÉQ. ID n° 2, en particulier dans laquelle le segment L du génome comprend une séquence nucléotidique au moins 95 % identique aux nucléotides 2706-14865 de la SÉQ. ID n° 2, en particulier dans laquelle le segment L du génome comprend la séquence nucléotidique constituée des nucléotides 2706-14865 de la SÉQ. ID n° 2, en particulier dans laquelle le segment S du génome comprend une séquence nucléotidique au moins 90 % identique aux nucléotides 2706-4377 de la SÉQ. ID n° 1 ;
et/ou dans laquelle le segment S du génome comprend une séquence nucléotidique au moins 95 % identique aux nucléotides 2706-4377 de la SÉQ. ID n° 1, en particulier dans laquelle le segment S du génome comprend la séquence nucléotidique constituée des nucléotides 2706-4377 de la SÉQ. ID n° 1.

7. PRV de la FHCC selon l'une quelconque des revendications 1 à 6, dans laquelle le segment S du génome comprend un cadre de lecture ouvert (ORF) de la nucléoprotéine du virus FHCC et un ORF hétérologue, en particulier dans laquelle l'ORF hétérologue code une partie d'un GPC du virus FHCC, ou dans laquelle l'ORF hétérologue code une protéine fluorescente, et/ou dans laquelle l'ORF de la nucléoprotéine et l'ORF hétérologue sont dans le cadre et sont séparés par la séquence codante pour un peptide 2A autoclivant, en particulier dans laquelle le peptide 2A comprend un peptide 2A (P2A) du teschovirus-1 porcin (PTV1), un peptide 2A (F2A) du virus de la fièvre aphteuse (FMDV), un peptide 2A (E2A) du virus de la rhinite équine A (ERAV) ou un peptide 2A (T2A) du virus de *Thosea asigna* (TaV).

8. Procédé de production d'une PRV de la FHCC, comprenant :
la transfection d'une cellule hôte avec :
un plasmide comprenant une copie antigénomique d'un segment L du virus FHCC ;
un plasmide comprenant une copie antigénomique d'un segment S du virus FHCC ;
un plasmide codant une glycoprotéine du virus FHCC (GPC) ;
un plasmide codant une nucléoprotéine du virus FHCC ;
un plasmide codant une protéine L du virus FHCC ; et
la mise en culture des cellules pendant suffisamment de temps pour produire une PRV de la FHCC comprenant un segment L du génome du virus FHCC, un segment S du génome du virus FHCC, le GPC, ou une partie de celui-ci, la nucléoprotéine et la protéine L.

9. Procédé selon la revendication 8, comprenant en outre une collecte de la PRV de la FHCC à partir du surnageant de la culture cellulaire, en particulier comprenant en outre une amplification de la PRV de la FHCC dans une lignée cellulaire sensible exprimant le GPC du virus FHCC en trans.

10. Procédé selon la revendication 8 ou 9, dans lequel le virus FHCC est la souche africaine IbAr10200, ou dans lequel le virus FHCC est la souche asiatique (Oman1998) ou la souche européenne (Turkey2004) du virus FHCC.

11. Procédé selon l'une quelconque des revendications 8 à 10, dans lequel le segment L du génome code une protéase de domaine de tumeur ovarienne virale (vOTU) comprenant une ou plusieurs mutations, les une ou plusieurs mutations perturbant l'activité désubiquitinase de la vOTU et/ou l'activité du gène stimulé par l'interféron (ISG15), en particulier dans lequel les une ou plusieurs mutations comprennent une mutation Q16R, numérotée en référence à la SÉQ. ID n° 8, et dans lequel l'activité désubiquitinase de la vOTU est perturbée, et/ou dans lequel les une ou plusieurs mutations comprennent ou comprennent en outre au moins l'une de I13R/E/K, V18I, C40A/S/R, P77D/T, T120L, E128V et A129R/G, numérotées en référence à la SÉQ. ID n° 8.

12. Procédé selon l'une quelconque des revendications 8 à 11, dans lequel le plasmide comprenant une copie antigénomique du segment S et le plasmide comprenant une copie antigénomique du segment L comprennent en outre un promoteur T7 fonctionnellement lié à l'extrémité 5' de la copie antigénomique et un ribozyme du virus de l'hépatite delta à l'extrémité 3' de la copie antigénomique.

13. Procédé selon l'une quelconque des revendications 8 à 12, dans lequel :
(i) le plasmide comprenant une copie antigénomique du segment L comprend une séquence nucléotidique qui est au moins 90 % identique ou au moins 95 % identique à la SÉQ. ID n°2 ;
(ii) le plasmide comprenant une copie antigénomique du segment S comprend une séquence nucléotidique qui est au moins 90 % identique ou au moins 95 % identique à la SÉQ. ID n° 1 ;
(iii) le plasmide codant le GPC du virus FHCC est au moins 90 % ou au moins 95 % identique à la SÉQ. ID n° 5 ;
(v) le plasmide codant la nucléoprotéine du virus FHCC comprend une séquence nucléotidique qui est au moins 90 % ou au moins 95 % identique à la SÉQ. ID n° 3 ; et/ou
(vi) le plasmide codant la protéine L du virus FHCC comprend une séquence nucléotidique qui est au moins 90 % ou au moins 95 % identique à la SÉQ. ID n° 4, en particulier dans lequel :
(i) la séquence nucléotidique du plasmide comprenant une copie antigénomique du segment L comprend la SÉQ. ID n° 2 ;
(ii) la séquence nucléotidique du plasmide comprenant une copie antigénomique du segment S comprend la SÉQ. ID n° 1 ;
(iii) la séquence nucléotidique du plasmide codant le GPC du virus FHCC comprend la SÉQ. ID n° 5 ;
(iv) la séquence nucléotidique du plasmide codant la nucléoprotéine du virus FHCC comprend la SÉQ. ID n° 3 ; et/ou
(v) la séquence nucléotidique du plasmide codant la protéine L du virus FHCC comprend la SÉQ. ID n° 4.

14. Composition immunogène comprenant la PRV de la FHCC selon l'une quelconque des revendications 1 à 7, et un support pharmaceutiquement acceptable, en particulier comprenant en outre un adjuvant.

15. Composition immunogène selon la revendication 14, destinée à une utilisation dans une méthode visant à provoquer une réponse immunitaire contre le virus FHCC ou une protéine virale du virus FHCC chez un sujet ou à immuniser un sujet contre l'infection par le virus FHCC, comprenant l'administration au sujet d'une quantité efficace de la composition immunogène,
en particulier dans laquelle la protéine virale est une nucléoprotéine du virus FHCC ou une protéine L du virus FHCC,
et/ou le sujet étant humain,
et/ou une seule dose de la composition immunogène étant administrée au sujet,
et/ou la composition immunogène étant administrée par voie intraveineuse, intramusculaire ou sous-cutanée,
et/ou la méthode induisant une réponse immunitaire à plus d'une souche du virus FHCC.
